# EUROPEAN PATENT SPECIFICATION

(11) **EP 1 934 598 B1**
(45) Date of publication and mention of the grant of the patent: **22.09.2010**
(21) Application number: 06794763.0
(22) Date of filing: 13.10.2006
(51) Int. Cl.: G01N 33/50, C12N 5/10, C12N 15/11, C12Q 1/18, C12Q 1/68, A61K 39/02, A61K 39/118, A61K 31/7048

(54) **DIAGNOSIS OF OBLIGATE INTRACELLULAR PATHOGENS**
DIAGNOSE OBLIGATER INTRAZELLULÄRER KRANKHEITSERREGER
DIAGNOSTIC D'INFECTIONS A PATHOGENES INTRACELLULAIRES OBLIGATOIRES

(30) Priority: 14.10.2005 US 726158 P; 19.10.2005 GB 0521290; 02.12.2005 US 741457 P; 06.01.2006 GB 0600244
(43) Date of publication of application: 25.06.2008
(73) Proprietor: Petyaev, Ivan Mikhailovich, 7 Hills Avenue Cambridge CB1 7UY (GB)
(72) Inventor: AKHATOVNA ZIGANGIROVA, Nailya, Moscow, 123308 (RU); PETYAEV, Ivan Mikhailovich, Cambridge Cambridgeshire CB1 7UY (GB)
(74) Representative: Sutcliffe, Nicholas Robert
(86) International application number: PCT/GB2006/003817
(87) International publication number: WO 2007/042827

(56) References cited:
- WO-A-03/019196
- WO-A2-02/18635
- WO-A2-03/077729
- APFALTER P ET AL: "Application of blood-based polymerase chain reaction for detection of Chlamydia pneumoniae in acute respiratory tract infections." EUROPEAN JOURNAL OF CLINICAL MICROBIOLOGY & INFECTIOUS DISEASES : OFFICIAL PUBLICATION OF THE EUROPEAN SOCIETY OF CLINICAL MICROBIOLOGY AUG 2001, vol. 20, no. 8, August 2001 (2001-08), pages 584-586, XP002427089 ISSN: 0934-9723
- TERAJIMA MASANORI ET AL: "Quantitation of antibody-bound and unbound Sin Nombre virus in the plasma of patient with hantavirus pulmonary syndrome." JOURNAL OF VIROLOGICAL METHODS 30 JUN 2003, vol. 110, no. 2, 30 June 2003 (2003-06-30), pages 159-162, XP002427902 ISSN: 0166-0934
- NAKAJIMA HIROFUMI ET AL: "Anti-viral actions and viral dynamics in the early phase of three different regimens of interferon treatment for chronic hepatitis C: differences between the twice-daily administration of interferon-beta treatment and the combination therapy with interferon-alpha plus ribavirin." ACTA MEDICA OKAYAMA OCT 2003, vol. 57, no. 5, October 2003 (2003-10), pages 217-225, XP002427803 ISSN: 0386-300X
- PODSIADLY E ET AL: "Presence of Chlamydia pneumoniae in patients with and without atherosclerosis" EUROPEAN JOURNAL OF CLINICAL MICROBIOLOGY & INFECTIOUS DISEASES, SPRINGER-VERLAG, BE, vol. 24, no. 8, 1 August 2005 (2005-08-01), pages 507-513, XP019355429 ISSN: 1435-4373
- DAXBOECK F ET AL: "Bacteremia with Mycoplasma hominis and Ureaplasma urealyticum in patients undergoing hysterectomy." EUROPEAN JOURNAL OF CLINICAL MICROBIOLOGY & INFECTIOUS DISEASES : OFFICIAL PUBLICATION OF THE EUROPEAN SOCIETY OF CLINICAL MICROBIOLOGY OCT 2003, vol. 22, no. 10, October 2003 (2003-10), pages 608-611, XP002427804 ISSN: 0934-9723
- APFALTER PETRA ET AL: "No evidence of involvement of Chlamydia pneumoniae in severe cerebrovascular atherosclerosis by means of quantitative real-time polymerase chain reaction." STROKE; A JOURNAL OF CEREBRAL CIRCULATION SEP 2004, vol. 35, no. 9, September 2004 (2004-09), pages 2024-2028, XP002427091 ISSN: 1524-4628
- KOVACIC R ET AL: "Search for the presence of six Mycoplasma species in peripheral blood mononuclear cells of subjects seropositive and seronegative for human immunodeficiency virus." JOURNAL OF CLINICAL MICROBIOLOGY JUL 1996, vol. 34, no. 7, July 1996 (1996-07), pages 1808-1810, XP002427805 ISSN: 0095-1137
- PAAVONEN J ET AL: "Chlamydia trachomatis: impact on human reproduction." HUMAN REPRODUCTION UPDATE 1999 SEP-OCT, vol. 5, no. 5, September 1999 (1999-09), pages 433-447, XP002427806 ISSN: 1355-4786
- MADICO G ET AL: "Touchdown enzyme time release-PCR for detection and identification of Chlamydia trachomatis, C. pneumoniae, and C. psittaci using the 16S and 16S-23S spacer rRNA genes." JOURNAL OF CLINICAL MICROBIOLOGY MAR 2000, vol. 38, no. 3, March 2000 (2000-03), pages 1085-1093, XP002427092 ISSN: 0095-1137 cited in the application
- DATABASE EMBL [Online] 16 February 1993 (1993-02-16), "major outer membrane protein=variable domain IV region [Chlamydia pneumoniae, Genomic, 170 nt]." XP002427095 retrieved from EBI accession no. EMBL:S50607 Database accession no. S50607
- BLANCHARD A ET AL: "DETECTION OF UREAPLASMA UREALYTICUM BY POLYMERASE CHAIN REACTION IN THE UROGENITAL TRACT OF ADULTS, IN AMNIOTIC FLUID, AND IN THE RESPIRATORY TRACT OF NEWBORNS" CLINICAL INFECTIOUS DISEASES, THE UNIVERSITY OF CHICAGO PRESS, CHICAGO, IL, US, vol. 17, no. SUPPL 1, August 1993 (1993-08), pages S148-S153, XP009081648 ISSN: 1058-4838 cited in the application

## Description

The present invention relates to the detection of obligate intracellular or membrane associated pathogens, such as *Chlamydia, Mycoplasma and Ureaplasma* species. This may be useful, for example, in the diagnosis and treatment of pathogen infections and diseases associated with pathogen infection.

At present, conventional assays for the diagnosis of pathogenic micro-organisms are based on samples of host cells, for example, obtained from swabs. These results obtained by these assays are highly variable and improved methods for the detection of pathogens would be desirable.

The present inventors have discovered that intracellular or membrane associated pathogen cells can be sensitively and reliably detected in acellular fractions of blood such as plasma and serum. These cells, which may be in the form of elementary bodies, or other cell forms, represent a distinct persistent form of the pathogen which is not reliably detectable by conventional means. Assays for the detection of these pathogen cells in the plasma and serum of patients may be useful in detecting pathogen infections and assessing pathogen-associated disease conditions.

An aspect of the invention provides a method for assessing an individual for infection with a persistent form of an obligate intracellular pathogenic bacterium as ser out in claim 1.

A method may comprise concentrating or enriching pathogen cells, such as elementary bodies, in the sample using a specific binding member to produce an enriched or concentrated fraction. A method for assessing an individual for infection with a persistent form of an obligate intracellular pathogenic bacterium may comprise:
contacting a blood sample from an individual with an specific binding member to isolate and/or purify a fraction of said sample, and;
determining the presence of nucleic acid from the pathogenic bacterium in said fraction.

The isolated/purified purified fraction may comprise an increased concentration of elementary bodies or other cells of the persistent form of the pathogenic bacterium, relative to the blood sample.

The presence of nucleic acid from the pathogenic bacterium in the sample or isolated fraction is indicative that the sample or the isolated and/or purified fraction comprises elementary bodies or other cells of the persistent form of the pathogenic bacterium and the individual is infected with infection with a persistent form of an obligate intracellular pathogenic bacterium.

Preferred specific binding molecules for use in aspects of the present invention include antibodies and fragments or derivatives thereof ('antibody molecules').

The isolation and/or purification of components and fractions of the sample using of a specific binding member such as an antibody may be performed by any appropriate technique and those skilled in the art are able to choose a suitable mode according to their preference and general knowledge.

The presence of an elementary body or other pathogen cell may be detected in the acellular fraction of a blood sample (i.e. in the plasma or serum).

The acellular fraction may be comprised within a whole blood sample or may be separated from the whole blood sample prior to detection to produce an acellular blood sample (i.e. a plasma or serum sample). A method as described herein may comprise providing an acellular sample of blood obtained from the individual (i.e. an acellular blood sample).

An acellular blood sample is a sample of blood from an individual that does not contain blood cells of the individual i.e. it is devoid of red blood cells, white blood cells, platelets and other host cells found in the blood. An acellular blood sample may, for example, be a plasma sample, which comprises clotting factors, or a serum sample, which lacks clotting factors.

An acellular blood sample may be produced by removing blood cells from a sample of blood obtained from the individual. Blood cells may be removed from the blood sample or preparation by any convenient method. For example, cells may be removed by centrifugation, typically at 2,000g for 10 mins, or by filtration, for example via a column, a membrane, a filter, or other separation device.

Pathogen cells are single units of the pathogenic bacterium and include elementary bodies, reticulate bodies and other forms of the bacterium.

Persistent infections are infections in which the pathogen is not cleared by host responses or therapeutic treatment but remains in specific cells, or associated with their membrane, of infected individuals.

The individual assessed as described herein may be a mammal, preferably a human. The individual may be healthy, i.e. may show no symptoms of a pathogen infection or a pathogen associated disorder (i.e. asymptomatic), or may display one or more known symptoms associated with a pathogen infection or a pathogen associated disorder. In some embodiments, an individual may be suspected of having or being at risk of developing a pathogen infection or an associated disorder.

The sample may be contacted with a specific binding member which binds to a complex comprising one or more plasma/serum molecules to isolate and/or purify a fraction which comprises one or more said complexes.

The one or more plasma/serum molecules may be antibodies and the complex may be an immunocomplex. The specific binding member may bind to immunoglobulin and thus to immunocomplexes in the sample. Suitable specific binding members include protein A, or anti-Ig, anti-IgA, anti-IgM antibodies, or other immunoglobulin or immune complex binding/capturing molecules.

The one or more plasma/serum molecules may be serum lipoproteins or lipopolysaccaride binding proteins (LBP). The specific binding member may bind to these serum lipoproteins or lipopolysaccaride binding proteins, for example anti-lipoprotein or anti-LBP antibodies.

A pathogen cell is a persistent form of an obligate intracellular pathogenic bacterium. Examples include bacteria of a Chlamydia species, such as *C. pneumoniae* or *C. trachomatis,* or a Mycoplasmataceae species, for example a mycoplasma such as *Mycoplasma genitalium,* or a ureaplasma such as *Ureaplasma urealyticum.*

The cell may, for example, be a pathogen of the respiratory tract, such as *C. pneumoniae* or a pathogen of the reproductive, or urinary tract, such as *C. trachomatis, Mycoplasma genitalium* or *Ureaplasma urealyticum.*

For example, a method for assessing an individual for a Chlamydia infection may comprise:
determining the presence of a Chlamydia cell in a blood sample obtained from the individual.

The Chlamydia cell may be a *C. pneumoniae* or *C. trachomatis* cell. In embodiments in which the pathogen is *C. pneumoniae,* the blood sample is preferably an acellular blood sample as described herein.

A method for assessing an individual for a Mycoplasmataceae infection may comprise:
determining the presence of a Mycoplasmataceae cell in a blood sample obtained from the individual.

A Mycoplasmataceae cell may include a mycoplasma or ureaplasma cell. A mycoplasma cell may be a *Mycoplasma genitalium* cell. A ureaplasma cell may be *Ureaplasma urealyticum* cell.

Methods described herein may be useful in the assessment of pathogen-associated disorders or conditions.

A method of assessing an individual for a pathogen-associated disorder may comprise:
determining the presence of a pathogen cell in a blood sample obtained from the individual.

The presence of a pathogen cell may be indicative of the presence of a disorder or condition associated with the pathogen.

Assessing an individual for a pathogen-associated disorder may include diagnosing a pathogen-associated disorder in the individual; determining the presence of a pathogen-associated disorder in the individual; or detecting a pathogen-associated disorder in the individual. In other embodiments, the severity or prognosis of a pathogen-associated disorder may be assessed in an individual or the risk that an individual will in the future suffer from a pathogen-associated disorder may be determined.

Diseases associated with *C. pneumoniae* may include respiratory, joint, cerebral or vascular conditions, including, for example, atherosclerotic conditions. An atherosclerotic condition may include a cardiovascular disorder such as atherosclerosis, ischaemic (coronary) heart disease, myocardial ischaemia (angina), myocardial infarction, aneurismal disease, atheromatous peripheral vascular disease, aortoiliac disease, chronic and critical lower limb ischaemia, visceral ischaemia, renal artery disease, cerebrovascular disease, stroke, atherosclerotic retinopathy, thrombosis and aberrant blood clotting, and hypertension. Such conditions may be medical or veterinary conditions

Diseases associated with *C. trachomatis* include for example chronic inflammatory diseases of the vagina, cervix, endometrium, fallopian tubes, prostate, bladder, urethra and reproductive dysfunction, or joint conditions.

Diseases associated with mycoplasma and ureaplasma include pneumonia and other respiratory disorders, chronic inflammatory diseases of the uterus, vagina, cervix, endometrium, fallopian tubes, prostate, bladder, urethra and reproductive dysfunction.

An individual suitable for testing using the present methods may show symptoms of acute genital infection. The present methods may confirm the presence of infection by a pathogen such as *Chlamydia trachomatis, Mycoplasma genitalium,* or *Ureaplasma urealyticum (spp.)* in patients diagnosed positively in a conventional PCR swab or urea test, or may identify the presence of infection in patients diagnosed negatively in a conventional PCR swab or urea test.

Other individuals suitable for testing as described herein may show no symptoms of acute genital infection but may display symptoms of chronic inflammatory diseases of the uterus, fallopian tubes, prostate, bladder, urethra, etc. These patients are typically negative in conventional PCR swab or urea tests. Positive detection of a pathogen such as *Chlamydia trachomatis, Mycoplasma genitalium, Ureaplasma urealyticum (spp.)* using the present methods would confirm the presence of chronic infection.

Other individuals suitable for testing as described herein may have infertility problems in which the presence of chronic asymptomatic inflammation in the reproductive system cannot be excluded. Pathogens such as *Chlamydia trachomatis, Mycoplasma genitalium, Ureaplasma urealyticum (spp.)* have been associated with miscarriage and the detection of infection by the present methods may be useful in treating infertility problems.

Methods described herein may also be useful in monitoring and verifying the elimination of pathogen infections following treatment and preventing the transmission of pathogen infections, for example by the transfusion of blood or the use of blood products from an individual who is negative for pathogen infection by conventional tests but positive by the present methods.

Preferably, the presence of a pathogen cell, such as an elementary body, in the sample is assessed by concentrating, isolating, and/or purifying the pathogen cells in the sample, for example by producing an isolated and/or purified fraction which is enriched for the elementary body or other pathogen cell, prior to detection.

In some embodiments, the sample is concentrated by isolating, purifying and/or concentrating complexes in the sample which comprise elementary bodies and other pathogen cells, or a fraction of the sample comprising such complexes. The presence of the pathogen cells in the isolated, purified and/or concentrated complexes may then be determined.

A complex comprising the pathogen cells, such as elementary bodies, or other forms of pathogen cells, may further comprise one or more plasma/serum molecules, such as immunoglobulin molecules, serum lipoproteins, lipopolysaccaride binding proteins or other bacteria-binding molecules present in the serum and/or plasma. For example, the elementary bodies or other pathogen cells may be comprised in an immunocomplex with one or more antibodies. Cells may be concentrated by isolating, purifying and/or concentrating the immunocomplexes from said sample, for example using an immunoglobulin binding agent.

Many techniques for isolating and/or purifying complexes are known in the art and may be used to concentrate the elementary bodies or other pathogen cells. These include physical and chemical/biochemical techniques and combinations thereof. For example, the sample may be contacted with an immunoglobulin-binding agent, such as protein A or an anti-Ig antibody, and unbound material removed or washed away. Immunocomplexes bound to the agent may be eluted using routine techniques or tested for the presence of pathogen cells directly.

Alternatively, the sample may be subjected to filtration, for example using a silico-gel column, or other suitable filter with a pore size suitable for retention of pathogen cells and/or complexes thereof.

The presence of an elementary body or other pathogen cell in a sample or a concentrated fraction thereof may be determined by any convenient means. For example, an immunochemical assay (for example, ELISA), or nucleic acid assay (for example, PCR), cell culture method, or animal test may be performed.

In some embodiments, a cell culture assay may be used to detect the presence of a pathogen cell in the sample.

The sample may, for example, be cultured under conditions suitable for growth of a pathogen and the growth of pathogen cells in the sample determined.

Techniques for the cell culture and identification of pathogenic micro-organisms such as *C. pneumoniae, C. trachomatis, Mycoplasma genitalium* and *Ureaplasma urealyticum,* are well known in the art.

In other embodiments, a non-human animal model may be used to detect the presence of a pathogen cell in the sample.

An animal model, for example a rodent, may be inoculated with said sample and the presence of pathogen infection in said animal model determined. The presence of infection in the animal is indicative of the presence of a pathogen cell in the sample.

In other embodiments, an immunochemical assay (for example, ELISA) may be used to detect the presence of pathogen cell antigens in the sample. For example, a method of determining the presence of a pathogen cell in a blood sample may comprise;
contacting the sample with an antibody which specifically binds to the pathogen cell; and,
determining binding of said antibody.

An antibody which specifically binds to a pathogen cell may bind to an antigen which is characteristic of the pathogen cell and may not show any significant binding to cells of other pathogen species. Antibodies which specifically bind pathogens such as *C. pneumoniae, C. trachomatis, Mycoplasma genitalium* and *Ureaplasma urealyticum* are well known in the art.

Binding of antibody molecules may be determined by any appropriate means. Tagging with individual reporter molecules is one possibility. The reporter molecules may directly or indirectly generate detectable, and preferably measurable, signals. The linkage of reporter molecules may be direct or indirect, covalent, e.g. via a peptide bond, or non-covalent. Linkage via a peptide bond may be as a result of recombinant expression of a gene fusion, encoding antibody and reporter molecule. For example, the antibody may be labelled with a fluorophore such as FITC or rhodamine, a radioisotope, or a non-isotopic-labelling reagent such as biotin or digoxigenin; antibodies containing biotin may be detected using "detection reagents" such as avidin conjugated to any desirable label such as a fluorochrome. In some embodiments, an additional antibody may be used to detect the binding of the first antibody.

The mode of determining binding is not a feature of the present invention and those skilled in the art are able to choose a suitable mode according to their preference and general knowledge.

Suitable approaches include immunohistochemical staining, immunocytochemical staining, Western Blotting, immunofluorescence, enzyme linked immunosorbent assays (ELISA), radioimmunoassays (RIA), immunoradiometric assays (IRMA) and immunoenzymatic assays (IEMA), including sandwich assays using monoclonal and/or polyclonal antibodies. All of these approaches are well known in the art.

An antibody for use in a method described herein may be immobilised or non-immobilised i.e. free in solution.

In preferred embodiments, a nucleic acid assay is used to detect the presence of nucleic acid, for example DNA or RNA, from the pathogen in the sample.

In some preferred embodiments, nucleic acid may be isolated or extracted from the sample. Preferably, nucleic acid is isolated or extracted non-specifically i.e. all nucleic acid in the sample is purified and/or removed from other components of the sample, such as proteins. The presence of nucleic acid from the pathogen in the isolated or extracted nucleic acid may then be determined.

A method may, for example, comprise;
isolating and/or purifying complexes, such as immunocomplexes, from a blood sample obtained from an individual,
isolating and/or extracting DNA from the complexes, and;
determining the presence of pathogen nucleic acid in the extracted nucleic acid.

Many suitable methods of extracting nucleic acid from a sample are known in the art and be employed, including silica-gel membrane columns e.g. QIAamp DNA Blood Mini Kit (QIAGen), Chelex chelating resin (Chelex 100, Sigma), or High Pure PCR template preparation kit (BM kit; Boehringer) etc. DNA may also be extracted by a conventional proteinase K phenol-chloroform protocol. RNA may be, for example, be conveniently isolated using the Trizol method (Invitrogen).

Techniques and protocols for manipulation of nucleic acid, including the extraction, isolation and purification of nucleic acid are described in detail in Current Protocols in Molecular Biology, Ausubel et al. eds. John Wiley & Sons, 1992, and Molecular Cloning: a Laboratory Manual: 3rd edition, Russell et al., 2001, Cold Spring Harbor Laboratory Press.

The presence of pathogen nucleic acid in the sample may be determined, optionally following nucleic acid extraction, using any suitable technique, including nucleic acid amplification (NAA) techniques and non- nucleic acid amplification techniques, including DNA probe hybridisation techniques such as Northern and Southern blotting.

In some preferred embodiments, the presence of pathogen nucleic acid is determined by a nucleic acid amplification technique, such as the polymerase chain reaction (PCR).

PCR comprises repeated cycles of denaturation of template nucleic acid, annealing of primers to template, and elongation of the primers along the template. PCR is well-known in the art and is described for example in "PCR protocols; A Guide to Methods and Applications", Eds. Innis et al, 1990, Academic Press, New York, Mullis et al, Cold Spring Harbor Symp. Quant. Biol., 51:263, (1987), Ehrlich (ed), PCR technology, Stockton Press, NY, 1989, and Ehrlich et a1, Science, 252:1643-1650, (1991)). The number of cycles, the respective conditions of the individual steps, the composition of reagents within the reaction tube, or any other parameter of the reaction set-up may be varied or adjusted by the skilled person, depending on the circumstances. Additional steps (such as initial denaturing, hot-start, touchdown, enzyme time release PCR, replicative PCR) may also be employed.

Variations of PCR include nested PCR, in which a second amplification is performed using a second pair of primers derived from sequences within the first amplification product, and RT-PCR (reverse-transcriptase PCR), in which RNA is reverse transcribed into cDNA and subsequently amplified by PCR.

Real-time PCR (or real-time reverse-transcriptase PCR) is a highly sensitive detection method that quantifies the initial amount of template (Arya et al, 2005, Expert Rev Mol Diagn. 5:209-219; Klein, 2002, Trends Mol Med 8:257-260). In some embodiments, real-time PCR might be employed.

Numerous variations and modifications of PCR and RT-PCR are known in the art and may be employed by the skilled person in performing the present methods. Chemicals, kits, materials and reagents are commercially available to perform PCR or RT-PCR reactions.

Other specific nucleic acid amplification techniques include strand displacement activation, the QB replicase system, the repair chain reaction, the ligase chain reaction, ligation activated transcription, SDA (strand displacement amplification) and TMA (transcription mediated amplification). For convenience, and because it is generally preferred, the term PCR is used herein in contexts where other nucleic acid amplification techniques may be applied by those skilled in the art. Unless the context requires otherwise, reference to PCR should be taken to cover use of any suitable nucleic amplification reaction available in the art.

The presence of pathogen nucleic acid may be determined by pathogen specific amplification. Pathogen specific amplification occurs only if nucleic acid from the pathogen is present in the sample. The production of an amplification product following pathogen specific amplification is indicative of the presence of a pathogen cell in the sample.

Pathogen specific amplification may typically be performed using one or more pathogen specific primers. For example, in PCR, one or both of the primers may hybridise specifically to pathogen nucleic acid, such that amplification products are only produced by the primers when pathogen nucleic acid is present in the sample.

The use of PCR for the specific detection of pathogens in a sample is well-known in the art and the skilled person is well versed in the design of suitable primers, reaction conditions etc. Suitable primers include oligonucleotides having about 10 or fewer codons (e.g. 6, 7 or 8), i.e. about 30 or fewer nucleotides in length (e.g. 18, 21 or 24). Generally, specific primers are upwards of 14 nucleotides in length, but need not be than 18-20. Various software is available for the design of primers, including Primer Express^{™}, Primer Premier 5^{™}, FastPCR^{™}, Primp^{™} and Oligo^{™}.

Various techniques for synthesizing oligonucleotide primers are well known in the art, including phosphotriester and phosphodiester synthesis methods. Custom-made oligonucleotide primers may be obtained from numerous commercial sources.

Based on available sequence information, it is routine for a person skilled in the art to design suitable primers which will only amplify nucleic acid from a specific pathogen, for example a Chlamydia such as *C. pneumoniae* or *C. trachomatis.*

The specificity of primers for nucleic acid sequences from a specific pathogen may be determined by comparing the sequences of the primers to genomic nucleic acid sequences from other pathogens, for example genomic nucleic acid sequences in sequence databases. Preferably, genomic nucleic acid sequences from phylogenically related pathogen are compared to the primer sequences. Primers which show little or no sequence identity to genomic nucleic acid sequences from phylogenically related pathogens may be predicted to show little or no hybridisation under the amplification conditions to such sequences and may be considered to be specific for the particular pathogen.

For example, the specificity of primers to nucleic acid from *Chlamydia pneumoniae,* may be determined by comparing the sequences of the primers to genomic nucleic acid sequences from other members of the Chlamydiaceae. For example, the specificity of the primers may be confirmed by aligning the targeted DNA sequence of primer set with DNA sequences of other bacteria and eucaryotic organisms in GenBank database.

Sequence comparisons may be performed using conventional sequence analysis software, such as BLAST, TBLASTN (which use the method of Altschul et al. (1990) J. Mol. Biol. 215: 405-410), psi-Blast (Nucl. Acids Res. (1997) 25 3389-3402) or FASTA (which uses the method of Pearson and Lipman (1988) PNAS USA 85: 2444-2448).

Alternatively, the specificity of primers to nucleic acid sequences from a specific pathogen, for example a *Chlamydia* cell such as *Chlamydia pneumoniae,* may be determined by performed nucleic acid amplification using the primers on genomic nucleic acid from a panel of micro-organisms. Primers which only amplify the genomic nucleic acid of the pathogen of interest may be specific for that pathogen.

The genomic sequences of many species are available and may be used to identify nucleic acid sequences which are unique to a specific pathogen and may be used as targets for the design of pathogen specific amplification primers. For example, the genome of *Chlamydia pneumoniae,* for example, has the database references gi15835535, NC_002491.1; gi15617929, NC_000922.1; gi33241335, NC_005043.1; and gi58021288 and NC_002179.2. The genomes of other pathogens are also available on public databases.

Primers specific for a pathogen hybridise under amplification conditions to target nucleic acid sequences within the genome of that pathogen which are unique to the pathogen and are not found in other pathogens, in particular in phylogenically related pathogens i.e. the primers do not hybridise under amplification conditions to nucleic acid sequences within the genome of pathogens other than the target pathogens. For example, *C. pneumoniae* specific primers do not hybridise under amplification conditions to sequences in the *C. trachomatis* genome and vice versa and *M. genitalium* specific primers do not hybridise under amplification conditions to sequences in the *U. urealyticum* genome.

For example, *Chlamydia pneumoniae* specific primers may, for example, amplify part of the 16S rRNA gene (database entry L06108.1 GI: 174111) or the variable domain IV (VDIV) region of the *ompA* gene of *C. pneumoniae.*

Primers directed at the 16S rRNA gene of *C. pneumoniae* may be selected from the group consisting of:
CPN90: 5' GGT CTC AAC CCC ATC CGT GTC GG 3'
CPN91: 5' TGC GGA AAG CTG TAT,TTC TAC AGT T 3' (Madico et al J Clin Microbiol. 2000 Mar;38(3):1085-93.)
Probe 553: 5'-CAAGTCCAGGTAAGGTCCTTCGCGTTGC-3'
Probe 557: 5'-TCCAGGTAAGGTCCTTCGCGTTGCATCG-3'
   5' TTACAAGCCTTGCCTGTAGG 3'
   5' GCGATCCCAAATGTTTAAGGC 3'
   5' TTATTAATTGATGGTACAATA 3'
   5' ATCTACGGCAGTAGTATAGTT 3'
CpnA 5' TGA CAA CTG TAG AAA TAC AGC 3'
CpnB 5'CGC CTC TCT CCT ATA AAT 3'

Primers directed at the the variable domain IV (VDIV) region of the *ompA* gene of *C. pneumoniae* may be selected from the group consisting of:
Cpn5P: 5' CCA ATA TGC ACA GTC CAA ACC TAA AA 3'
Cpn3P: 5' CTA GAT TTA AAC TTG TTG ATC TGA CAG 3'
Cpn5N 5' CTC TGT AAA CAA ACC GGG C 3'
Cpn3N 5' GAT CTG ACA GGA AAC AAT TTG CAT 3'
Cloned *Pst*I HL1 5' GTT GTT CAT GAA GGC CTA CT 3'
   HR1 5' TGC ATA ACC TAC GGT GTG TT 3'
*ompA* (Nested) CP1 5'.TTA CAA GCC TTG.CCT GTA GG 3'
   CP2 5' GCG ATC CCA AAT GTT TAA GGC 3'
   CPC 5' TTA TTA ATT GAT GGT ACA ATA 3'
   CPD 5' ATC TAC GGC AGT AGT ATA GTT 3'

*Chlamydia trachomatis* specific primers may, for example, amplify part of the cryptic plasmid (see, for example, X06707.2 GI:4691224) of this pathogen.

Suitable *Chlamydia trachomatis* specific primers include;
CP 24 - 5' GGGATTCCTGTAACAACAAGTCAGG 3'
CP 27 - 5' CCTCTTCCCCAGAACAATAAGAAC 3'.

Mycoplasma genitalium specific primers may, for example, amplify all or part of the 140 kDa adhesion protein gene, MgPa (Jensen JS et al J. Clin. Microbiol. 1991 Jan; 29(1): 46-50; M31431.1 GI:150157).

Suitable *Mycoplasma genitalium* specific primers include
MgPa-1 - 5' AGTTGATGAAACCTTAACCCCTTGG 3'
MgPa-3 - 5' CCGTTGAGGGGTTTTCCATTTTTGC 3'

*Ureaplasma urealytica* specific primers may, for example, amplify all or part of the urease gene of *Ureaplasma* spp. (Blanchard A et al Clin Infect Dis. 1993 Aug;17 Suppl 1:S148-53; AF085724.2 GI:9967025) Suitable *Ureaplasma urealytica* specific primers include:
U1 - 5' GATGGTAAGTTAGTTGCTGAC 3'
U2 - 5' ACGACGTCCATAAGCAACT 3'.

Following pathogen-specific amplification of the sample, the presence of amplification products may be detected. The presence of amplification products following pathogen specific amplification is indicative of the presence of pathogen cells in the sample.

Numerous techniques are known in the art for the detection of the nucleic acid amplification products. Suitable techniques for example, include gel- or capillary electrophoresis, chromatography, array hybridisation, Strand Displacement Amplification (SDA), or sequencing. Detection and/or isolation of the amplified nucleic acid product might require labelling or tagging the nucleic acid fragment, either simultaneously or subsequently to the actual amplification reaction.

Other aspects of the invention relate to the provision of kits for detecting pathogen cells in a cellular or acellular blood sample, for example for the detection of pathogen infection or assessment of a disease associated with the pathogen.

Pathogen specific primers as described above may form part of such a kit e.g. in a suitable container such as a vial in which the contents are protected from the external environment.

A kit for detecting pathogen infection or assessing an individual for a disease associated with a pathogen infection may comprise:
one or more primers specific for an obligate intracellular pathogenic bacterium
reagents for amplifying pathogen specific nucleic acid from a serum sample using the primers and;
detection reagents for detecting the products of amplifying the serum sample with the primers.

The detection reagents may comprise buffer solutions, wash solutions etc.

A kit may further comprise apparatus for the removal, handling and storage of blood samples and/or means for the removal of blood cells from the blood sample. Suitable means may include filtration devices.

A kit may comprise a specific binding member for isolating and/or purifying a fraction of a blood sample comprising elementary bodies of said persistent pathogen infection. Suitable specific binding members include immunoglobulin-binding agents for isolating and /or purifying immunocomplexes in a blood sample. Immunoglobulin-binding agents include protein A and anti-Ig antibodies. The immunoglobulin-binding agent may be immobilised on a solid support or matrix.

A kit may further comprise apparatus for isolating and/or purifying DNA for amplification by said primers. Suitable apparatus is well known in the art and described elsewhere herein.

The kit may also include instructions for use of the pathogen specific primers on a serum sample e.g. in a method of detecting a pathogen infection or disease associated with a pathogen, as described herein.

A method of isolating a cell of a persistent-form of an obligate intracellular pathogenic bacterium may comprise;
isolating and/or purifying a fraction of a blood sample obtained from an individual which comprises cells of said persistent form,
isolating and/or purifying said cell from said fraction.

The cell may be an elementary body of the persistent-form of the micro-organism.

The isolated elementary body or other cell may be from a persistent-form of obligate intracellular or membrane associated pathogenic micro-organism selected from the group consisting of *C. pneumoniae, C. trachomatis, Mycoplasma genitalium* or *Ureaplasma urealyticum.*

The isolated elementary body or other cell of the persistent form micro-organism may be capable of infecting a cell to produce a reticular body which is LPS deficient and antibiotic insensitive, relative to reference strains of said micro-organism. The isolated elementary body or other cell may also have atypical morphology of small intracellular inclusions.

Suitable reference strains of obligate intracellular or membrane associated pathogenic micro-organism include *C. pneumoniae* TW-183, AR-39, Kajaani 6, *C. trachomatis Bu434, Mycoplasma genitalium* G37 and M30and *Ureaplasma urealyticum* serotypes 1 to 10. These are available from commercial culture collections (e.g. European Collection of Cell Cultures (ECACC) Salisbury UK)

Elementary bodies or other cells of the persistent form micro-organism may be isolated and/or purified using conventional concentration techniques such as sorbents, filters or ultracentrifugation.

In some embodiments, the isolated elementary body or other cell may be from a persistent-form of *Chlamydia trachomatis* which is capable of infecting a cell to produce a reticular body which does not express Omp2 (60 kDa), Omp3 (.9 kDa) or PmpD, and expresses increased levels of Hsp60; and reduced levels of MOMP, relative to reference strains of *Chlamydia trachomatis.*

In some embodiments, the isolated elementary body or other cell may be from a persistent-form of *Chlamydia pneumoniae* which is capable of infecting a cell to produce a reticular body which does not express LPS and comprises inclusions of reduced size relative to reference strains of *Chlamydia pneumoniae.*

A method of producing a host cell infected with a persistent-form of an obligate intracellular pathogenic barcterium may comprise:
isolating and/or purifying a fraction of a blood sample obtained from an individual which comprises elementary bodies of said persistent form,
contacting a population of host cells with said fraction, and identifying a host cell in said population which comprises the persistent-form of the bacterium.

Suitable host cells for obligate intracellular pathogenic bacterium include cultured mammalian cells such as McCoy, HL, Hep-2, or HeLa cells.

Host cells identified as comprising the persistent-form of the bacterium, for example in the form of an intracellular reticular body, may be isolated and/or purified.

The identified host cells may be cultured further. Methods and techniques for the culture of mammalian cells are well-known in the art (see, for example, Basic Cell Culture Protocols, C. Helgason, Humana Press Inc. U.S. (15 Oct 2004) ISBN: 1588295451; Human Cell Culture Protocols (Methods in Molecular Medicine S.) Humana Press Inc., U.S. (9 Dec 2004) ISBN: 1588292223: Culture of Animal Cells: A Manual of Basic Technique, R. Freshney, John Wiley & Sons Inc (2 Aug 2005) ISBN: 0471453293, Ho WY et al J Immunol Methods. (2006) 310:40-52). Standard mammalian cell culture conditions may be employed, for example 37°C, 21% Oxygen, 5% Carbon Dioxide. Media is preferably changed every two days and cells allowed to settle by gravity.

Obligate intracellular pathogenic bacterium may be selected from the group consisting of *C. pneumoniae, C. trachomatis, Mycoplasma genitalium* and *Ureaplasma urealyticum,* which are described in more detail above.

A host cell may comprise a persistent-form obligate intracellular or membrane associated pathogenic micro-organism which is obtainable by a method described above.

The host cell may comprise one or more cells, in the form of reticular bodies, of a persistent-form of an obligate , intracellular pathogenic bacterium which has reduced antibiotic sensitivity relative to reference strains of the micro-organism.

The reticular bodies of the persistent-form of the obligate intracellular pathogenic bacterium may have reduced or absent lipopolysaccaride relative to reference strains of said bacterium.

The reticular bodies of the persistent-form of the obligate intracellular pathogenic bacterium may have altered gene expression relative to reference strains of said micro-organism. In some embodiments, the reticular bodies may not express certain genes which are typically expressed by reference strains, for example in an acute form of the infection, and may express genes which are not expressed by reference strains. The reticular bodies may also possess over-, under- or other abnormal expression of other genes, relative to the reference strains. For example, the reticular bodies of the persistent-form of *Chlamydia trachomatis* do not express Omp2 (60 kDa), Omp3(9 kDa) or PmpD, and; express increased levels of Hsp60; and reduced levels of MOMP, relative to reference strains of *Chlamydia trachomatis.*

A method of screening for an anti-microbial compound may comprise;
contacting a host cell infected with the persistent-form of an obligate intracellular pathogenic bacterium as described herein with a test compound; and
determining the effect of said compound on the pathogenic bacterium in said host cells,
wherein a reduction in the amount of said pathogenic bacterium in said host cells relative to controls is indicative that the test compound is an anti-microbial compound.

Preferably, the test compound is non-toxic to the host cells. A concentration range for the test compound or preparation may be initially identified which is not toxic to the host cells. The host cells may then be contacted with concentrations of test compound within this range to determine the effect on the pathogenic micro-organism.

The precise format for performing the methods described herein may be varied by those of skill in the art using routine skill and knowledge.

Compounds which may be screened using the methods described herein may be natural or synthetic chemical compounds used in drug screening programmes. Extracts of plants, microbes or other organisms which contain several characterised or uncharacterised components may also be used.

Combinatorial library technology provides an efficient way of testing a potentially vast number of different compounds for ability to modulate an interaction. Such libraries and their use are known in the art, for all manner of natural products, small molecules and peptides, among others. The use of peptide libraries may be preferred in certain circumstances.

The amount of test compound or compound which may be added to a method of the invention will normally be determined by serial dilution experiments. Typically, from about 0.001 nM to 1 mM or more of putative inhibitor compound may be used, for example from 0.01 nM to l00µM, e.g. 0.1 to 50 µM, such as about 10 µM,

A method may comprise identifying the test compound as an anti-microbial compound. Such a compound may, for example, be useful in reducing or eliminating infection with a bacterium as described herein, for example in the treatment of an infection or infection associated condition, as described herein.

A test compound identified using one or more initial screens as having ability to reduce the amount of said pathogenic bacterium organism in said host cells relative to controls, may be assessed further using one or more secondary screens. A secondary screen may, for example, involve testing for an effect on bacterial infection or a disorder associated with bacterial infection in an animal model.

The test compound may be isolated and/or purified or alternatively, it may be synthesised using conventional techniques of recombinant expression or chemical synthesis. Furthermore, it may be manufactured and/or used in preparation, i.e. manufacture or formulation, of a composition such as a medicament, pharmaceutical composition or drug. These may be administered to individuals for the treatment of a micro-organism infection or a disorder associated with micro-organism infection. Methods of the invention may thus comprise formulating the test compound in a pharmaceutical composition with a pharmaceutically acceptable excipient, vehicle or carrier for therapeutic application, as discussed further below.

Following identification of a compound which possesses anti-microbial activity, a method may further comprise modifying the compound to optimise the pharmaceutical properties thereof.

The modification of a 'lead' compound identified as biologically active is a known approach to the development of pharmaceuticals and may be desirable where the active compound is difficult or expensive to synthesise or where it is unsuitable for a particular method of administration, e.g. peptides are not well suited as active agents for oral compositions as they tend to be quickly degraded by proteases in the alimentary canal. Modification of a known active compound (for example, to produce a mimetic) may be used to avoid randomly screening large number of molecules for a target property.

Modification of a 'lead' compound to optimise its pharmaceutical properties commonly comprises several steps. Firstly, the particular parts of the compound that are critical and/or important in determining the target property are determined. In the case of a peptide, this can be done by systematically varying the amino acid residues in the peptide, e.g. by substituting each residue in turn. These parts or residues constituting the active region of the compound are known as its "pharmacophore".

Once the pharmacophore has been found, its structure is modelled according its physical properties, e.g. stereochemistry, bonding, size and/or charge, using data from a range of sources, e.g. spectroscopic techniques, X-ray diffraction data and NMR.

Computational analysis, similarity mapping (which models the charge and/or volume of a pharmacophore, rather than the bonding between atoms) and other techniques can be used in this modelling process.

In a variant of this approach, the three-dimensional structure of the anti-microbial compound is modelled. This can be especially useful where the compound changes conformation, allowing the model to take account of this in the optimisation of the lead compound.

A template molecule is then selected, onto which chemical groups that mimic the pharmacophore can be grafted. The template molecule and the chemical groups grafted on to it can conveniently be selected so that the modified compound is easy to synthesise, is likely to be pharmacologically acceptable, and does not degrade in vivo, while retaining the biological activity of the lead compound. The modified compounds found by this approach can then be screened to see whether they have the target property, or to what extent they exhibit it. Modified compounds include mimetics of the lead compound.

Further optimisation or modification can then be carried out to arrive at one or more final compounds for in vivo or clinical testing.

As described above, a compound identified and/or obtained using the present methods may be formulated into a pharmaceutical composition.

While it is possible for an active anti-microbial compound to be administered alone, it is preferable to present it as a pharmaceutical composition (e.g., formulation) comprising at least one active compound, as defined above, together with one or more pharmaceutically acceptable carriers, adjuvants, excipients, diluents, fillers, buffers, stabilisers, preservatives, lubricants, or other materials well known to those skilled in the art and optionally other therapeutic or prophylactic agents.

Pharmaceutical compositions comprising an anti-microbial compound as defined above, for example, an inhibitor admixed or formulated together with one or more pharmaceutically acceptable carriers, excipients, buffers, adjuvants, stabilisers, or other materials, as described herein, may be used in the methods described herein.

The term "pharmaceutically acceptable" as used herein pertains to compounds, materials, compositions, and/or dosage forms which are, within the scope of sound medical judgement, suitable for use in contact with the tissues of a subject (e.g., human) without excessive toxicity, irritation, allergic response, or other problem or complication, commensurate with a reasonable benefit/risk ratio. Each carrier, excipient, etc. must also be "acceptable" in the sense of being compatible with the other ingredients of the formulation.

Suitable carriers, excipients, etc. can be found in standard pharmaceutical texts, for example, Remington's Pharmaceutical Sciences, 18th edition, Mack Publishing Company, Easton, Pa., 1990.

The formulations may conveniently be presented in unit dosage form and may be prepared by any methods well-known in the art of pharmacy. Such methods include the step of bringing the active compound into association with a carrier which may constitute one or more accessory ingredients. In general, the formulations are prepared by uniformly and intimately bringing into association the active compound with liquid carriers or finely divided solid carriers or both, and then if necessary shaping the product.

Formulations may be in the form of liquids, solutions, suspensions, emulsions, elixirs, syrups, tablets, lozenges, granules, powders, capsules, cachets, pills, ampoules, suppositories, pessaries, ointments, gels, pastes, creams, sprays, mists, foams, lotions, oils, boluses, electuaries, or aerosols.

Conditions which are associated with pathogen infection may be treated and monitored using the methods described herein.

A method of treating a condition associated with pathogen infection may comprise;
administering an anti-microbial compound to the individual; and,
determining the presence of a pathogen cell in the acellular fraction of a blood sample obtained from the individual following said administration.

Administration of the anti-microbial compound to the individual may be repeated until pathogen cells are found to be absent from the acellular fraction of the blood sample.

Methods for the determination of the presence of a pathogen cell in the acellular fraction of a blood sample are described in more detail above.

The pathogen may be a persistent form of an obligate intracellular pathogenic bacterium. A method of treatment of persistent infection with.an obligate intracellular pathogenic bacterium in an individual may comprise:
i) administering an anti-microbial compound to the individual;
ii) obtaining a blood sample from the individual after said administration,
iii) contacting said blood sample with an specific binding member to isolate and/or purify a fraction of said samples, and;
(iii) determining the presence of nucleic acid from the pathogenic bacterium in said fraction of said sample
iv) repeating steps i) to iii) until no nucleic acid from the pathogenic bacterium is determined to be present in fraction from the sample.

Methods for the determination of the presence of nucleic acid from the pathogenic bacterium in said fraction of the sample are described in more detail above.

An anti-microbial compound kills or inhibits the growth of pathogenic bacteria such as Chlamydia spp or *Mycoplasmatacea* spp. Suitable anti-microbial compounds include erythromycin, rifalazil, roxithromycin, ofloxacin, clinafloxacin, ciprofloxacin, clindamycin, azithromycin, doxycycline, minocycline, tetracycline, moxifloxacin, rifampin, coarithromycin, grepafoaxcin, luvofloxacin, trovafloxacin, telithromycin, clarithromycin, quinolones and/or other fluoroquinolones, macrolides, ketolides, or from other groups of anti-microbial drugs.

A pathogen may include a Chlamydia spp such as *C. trachomatis* or *C. pneumoniae,* or a *Mycoplasmatacea* spp, for example a mycoplasma, such as *Mycoplasma genitalium,* or ureaplasma, such as *Ureaplasma urealyticum.*

Diseases associated with these pathogens are described in more detail above.

For example, a method of treating a sexually transmitted infection or an inflammatory disorder, such as Pelvic Inflammatory Disease, which is associated with pathogen infection may comprise;
administering an anti-microbial compound to the individual; and,
determining the presence of a pathogen cell in the acellular fraction of a blood sample obtained from the individual following said administration.

Sexually transmitted infections include infections with *C. trachometis* or a *Mycoplasmatacea* spp. Inflammatory disorders, such as Pelvic Inflammatory Disease, may be associated with infection with a pathogen such as *C. trachomatis* or a *Mycoplasmatacea* spp.

The methods described herein may be useful in reducing the number of false negative cases in the diagnosis of pathogens such as *C. trachomatis,* increasing the efficacy of anti-microbial treatment by increasing the probability of its completion and preventing the development of chronic persistent forms of the infection by assuring eradication of pathogens such as *C trachomatis.*

Atherosclerotic conditions may be treated and monitered using the methods described herein.

Atherosclerotic conditions are associated with *C. pneumoniae* infection, and, in particular, to catalytic antibodies known as 'abzymes' which oxidise lipid and are reactive with *C. pneumoniae.*

Effective treatment of atherosclerotic conditions may therefore require both treatment to reduce abzyme levels and treatment to reduce or eliminate underlying *C. pneumoniae* infection.

Detection of *C. pneumoniae* in blood as described herein may be useful in identifying individuals suitable for anti-microbial treatment, increasing the efficacy of anti-microbial treatment by increasing the probability of its completion and assuring eradication of *C. pneumoniae,* to prevent reappearance of anti-Chlamydia lipid oxidising abzymes and development of atherosclerotic conditions.

A method of treating an atherosclerotic condition in an individual may comprise;
i) administering an abzyme inhibitor and an anti-microbial compound to the individual;
ii) obtaining a blood sample from the individual after said administration,
iii) determining the level of abzyme activity in the acellular fraction of the blood sample,
   wherein said administration of the abzyme inhibitor to the individual is repeated until abzyme activity is substantially absent from the acellular fraction, and
iv) determining the presence or absence of *C. pneumoniae* elementary bodies or other cells from the acellular fraction of the blood sample using a method described above,
   wherein said administration of the anti-microbial compound to the individual is repeated until *C. pneumoniae* elementary bodies or other cells are absent from the acellular fraction of the blood sample.

An abzyme inhibitor is a compound that inhibits the lipid oxidation activity of catalytic antibodies, for example IgG molecules, in the serum or plasma. Catalytic antibodies that display lipid oxidation activity may be anti-Chlamydia antibodies.

Suitable abzyme inhibitors include azithromycin, lactolycopene, carotene, α-tocopherol, desferrioxamine mesylate, catechins such as EGCG, haem derivatives, penicillamine, tiopronin, trientine dihydrochloride, diethyldithiocarbamate, disodium/trisodium edetate, acetylsalicylic acid, edetic acid, ketolides, unithiol, α-tocopherol, mannitol, silidianin, ascorbic acid and other antioxidants effective at low pH.

Suitable anti-microbial compounds are described above.

In some embodiments, a single compound which has both anti-microbial activity and abzyme inhibition activity may be employed. Suitable compounds include azithromycin and telithromycin (ketek).

For example, a method of treating an atherosclerotic condition in an individual may comprise;
i) administering azithromycin, to the individual;
ii) obtaining a blood sample from the individual after said administration,
iii) determining the level of abzyme activity in the acellular fraction of the blood sample, and;
iv) determining the presence or absence of *C. pneumoniae* cells in the acellular fraction of the blood sample using a method described above,
   wherein said administration of azithromycin to the individual is repeated until abzyme activity and *C. pneumoniae* cells are absent from the acellular fraction of a blood sample obtained from the individual.

Abzyme inhibitors and anti-microbial compounds may be administered as pharmaceutical compositions. A pharmaceutical composition may include, in addition to the active abzyme inhibitor or anti-microbial compound, a pharmaceutically acceptable excipient, carrier, buffer, stabiliser or other material well known to those skilled in the art. Such materials should be non-toxic and should not interfere with the efficacy of the active ingredient. The precise nature of the carrier or other material will depend on the route of administration, which may be oral, or by injection, e.g. cutaneous, subcutaneous or intravenous.

Pharmaceutical compositions for oral administration may be in tablet, capsule, powder or liquid form. A tablet may include a solid carrier, such as gelatin, or an adjuvant. Liquid pharmaceutical compositions generally include a liquid carrier such as water, petroleum, animal or vegetable oils, mineral oil or synthetic oil. Physiological saline solution, dextrose or other saccharide solution or glycols such as ethylene glycol, propylene glycol or polyethylene glycol may be included.

For intravenous, cutaneous or subcutaneous injection, the active ingredient will be in the form of a parenterally acceptable aqueous solution which is pyrogen-free and has suitable pH, isotonicity and stability. Those of relevant skill in the art are well able to prepare suitable solutions using, for example, isotonic vehicles such as Sodium Chloride Injection, Ringer's Injection, or Lactated Ringer's Injection. Preservatives, stabilisers, buffers, antioxidants and/or other additives may be included, as required.

Administration of abzyme inhibitors and/or anti-microbial compounds is preferably in a "prophylactically effective amount" or a "therapeutically effective amount" (as the case may be, although prophylaxis may be considered therapy), this being sufficient to show benefit to the individual. The actual amount administered, and rate and time-course of administration, will depend on the nature and severity of what is being treated. Prescription of treatment, e.g. decisions on dosage etc, is within the responsibility of general practitioners and other medical doctors.

In some preferred embodiments, the abzyme inhibitors and/or anti-microbial compounds are administered to the individual periodically, for example, hourly, daily, weekly, biweekly or monthly. Periodic administration may continue until abzyme activity and/or pathogen cells are found to be reduced or eliminated from blood samples obtained from the individual.

Blood samples may be obtained from the individual periodically, for example daily, weekly, biweekly or monthly, while the individual is undergoing treatment with abzyme inhibitor and/or anti-microbial compound, and the level of abzyme activity and/or presence of pathogen cells in the samples determined.

The level of abzyme activity in the acellular fraction of a blood sample obtained from the individual may be determined by determining the antibody-mediated lipid oxidation activity in the fraction, in particular the lipid oxidation activity in the fraction which is mediated by anti-Chlamydia antibodies. For example, an antibody, preferably a Chlamydia binding antibody, from the serum or plasma fraction of the sample may be tested for its ability to oxidise lipid.

Lipid oxidation activity, including lipid peroxidation activity, may be determined by determining (for example by measuring or detecting) the oxidation of host lipid (i.e. lipid from the sample), lipid from a foreign antigen such as a Chlamydia cell, or lipid from another source, which may for example be added as part of an assay method.

Lipid oxidation may be determined by measuring the accumulation of products or by-products, such as co-oxidised coupled reporter molecules or the disappearance or consumption of substrates such as non-modified lipids or co-substrates such as oxygen.

Many methods for determining lipid peroxidation are known in the art and are suitable for use in accordance with the present invention. The precise mode of determining lipid oxidation is not a feature of the present invention and those skilled in the art are able to choose a suitable mode according to their preference and general knowledge.

Suitable methods are, for example, described in CRC Handbook of Methods for Oxygen Radical Research, CRC Press, Boca Raton, Florida (1985), Oxygen Radicals in Biological Systems. Methods in Enzymology, v. 186, Academic Press, London (1990); Oxygen Radicals in Biological Systems. Methods in Enzymology, v. 234, Academic Press, San Diego, New York, Boston, London (1994); and Free Radicals. A practical approach. IRL Press, Oxford, New York, Tokyo (1996)

In preferred embodiments, oxidation is determined by determining the production (i.e. the presence or amount) of a lipid oxidation product.

Oxidation products and/or intermediates of the lipids in which oxidation was initiated may be determined or oxidation products and/or intermediates may be determined of lipids in which oxidation is propagated.

A suitable lipid oxidation product may include aldehydes such as malondialdehyde (MDA), (lipid) peroxides, diene conjugates or hydrocarbon gases. Lipid oxidation products may be determined by any suitable method. For example, lipid peroxidation products may be determined using HPLC (Brown, R.K., and Kelly, F.J In: Free Radicals. A practical approach. IRL Press, Oxford, New York, Tokyo (1996), 119-131), UV spectroscopy (Kinter, M. Quantitative analysis of 4-hydroxy-2-nonenal. Ibid.,133-145), or gas chromatography-mass spectrometry (Morrow, J.D., and Roberts, L.J. F₂-Isoprostanes: prostaglandin-like products of lipid peroxidation. Ibid., 147-157).

The peroxidation of lipid may lead to an oxidation of proteins, carbohydrates, nucleic acids and other types of molecules. The products of such oxidation can also be used for indirect measurement of the activity of the abzymes. In addition, peroxidation may lead to changes in the properties of reporter molecules associated with propagating lipid oxidation. As described below, reporter molecules may be encapsulated in these lipids, for example as liposomes, and release of the reporter molecule from the liposome is indicative of oxidation.

Suitable reporter substances and molecules may include intact luminous bacteria, luminol, lucigenin, pholasin and luciferin. Such substances may, for example, be coupled to H₂O_{2/}O₂^{•⁻}/O₂- utilizing molecules such as peroxidase, esterase, oxidase, luciferase, catalase, superoxide dismutase, perylene, NAD⁺, and acridinium esters bis (trichlorophenyl) oxalate (Campbell A.K. Chemiluminescence. VCH, Ellis Horwood Ltd., England, 1988)

Other materials susceptible to free radical chain reactions may also be used to determine lipid oxidation. For example, lipid peroxidation, as a chain process, initiates and enhances the polymerisation of acrylamide. Lipid oxidation may thus be determined by the determining the co-polymerisation of ¹⁴C-acrylamide (Kozlov Yu P. (1968) Role of Free Radicals in normal and pathological processes. Doctorate thesis - MGU Moscow 1968)

Since lipid and lipoprotein peroxidation is a free radical mediated process, lipid oxidising abzymes may be measured by detection of these radicals. Radicals may be detected or determined using intrinsic low-level chemiluminescence (with or without sensitisors)(Vladimirov, Y.A., and Archakov, A.I. Lipid Peroxidation in Biological Membranes. Nauka, Moscow (1972); Vladimirov, Y.A. Intrinsic low-level chemiluminescence. In: Free Radicals. A practical approach. IRL Press, Oxford, New York, Tokyo (1996), 65-82), electron spin resonance (with spin trapping (Mason, R.P. In vitro and in vivo detection of free radical metabolites with electron spin resonance. In: Free Radicals. A practical approach. IRL Press, Oxford, New York, Tokyo (1996), 11-24) or without spin trapping (Petyaev, M.M. Biophysical approaches in the diagnosis of cancer. Medicina, Moscow (1972)) or other techniques well known in the art. Lipid oxidation may also be determined by determining the consumption of fatty acids or other substrates of this reaction.

In some preferred embodiments, the production of malondialdehyde (MDA) is determined, following reaction with 2-thiobarbituric acid (conventiently 1mM) by measuring absorbance at an appropriate wavelength such as 525 nm.

In some embodiments, the ability of an antibody from the serum or plasma fraction of the sample to bind to a Chlamydia cell, for example a *C. pneumoniae, C. psittaci,* or *C. trachomatis* cell may be determined.

The determination of abzyme activity is described in more detail in WO03/019196, WO03/0191 and WO03/017992

Treatment of the individual with the abzyme inhibitor may continue until the level of abzyme activity in the acellular fraction of a blood sample from the individual is determined to be zero or substantially zero. After the level of abzyme activity in the acellular fraction of a blood sample from the individual has been reduced to zero or substantially zero, treatment with the abzyme inhibitor may cease.

The presence or absence of a. *C*. *pneumoniae* cell in the acellular fraction of the blood sample may be determined using a method described above.

Treatment with the anti-microbial compound may continue until no *C. pneumoniae* cells are detected in the acellular fraction of a blood sample from the individual. After *C. pneumoniae* cells have 38 been eliminated from the acellular fraction of a blood sample, treatment with the anti-microbial compound may cease.

In some embodiments, the efficacy of an anti-microbial compound in treating persistant infection with an obligate intracellular pathogenic bacteriumicro-organis in an individual may be tested. A method may comprise:
i) obtaining a blood sample from the individual before and after administration of the anti-microbial compound,
(ii) contacting said blood samples with an specific binding member to isolate and/or purify a fraction of said samples, and;
(iii) determining the presence of nucleic acid from the pathogenic bacterium in said fractions of said samples,
   wherein a decrease in the amount of nucleic acid from the pathogenic bacterium in said fraction from the sample obtained after administration relative to the sample obtained before administration is indicative that the anti-microbial preparation is efficacious in treating the persistent infection.

Various further aspects and embodiments of the present invention will be apparent to those skilled in the art in view of the present disclosure.

Certain aspects and embodiments of the invention will now be illustrated by way of example and with reference to the figures and tables described below.

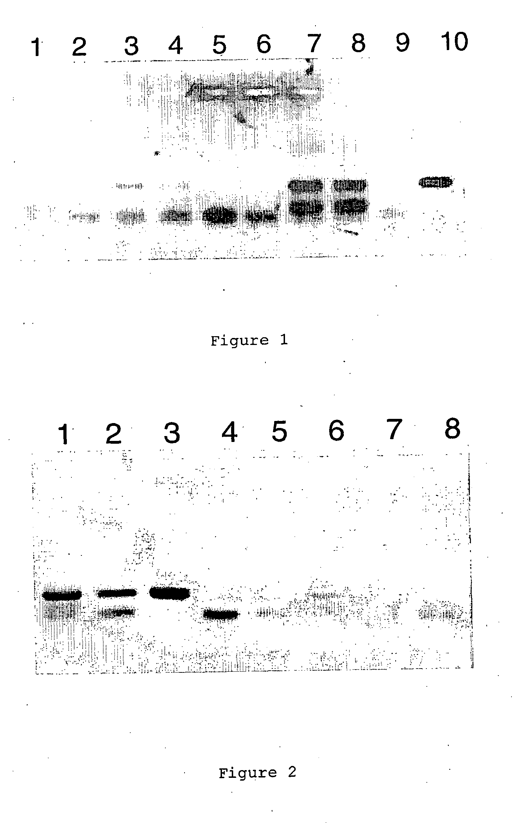

Figure 1 shows detection of Chlamydia pneumoniae DNA in CHD patients. Lanes 3 and 4 and lanes 7 and 8 show the use of a protein A concentration step whereas lanes 5 and 6 do not employ a protein A concentration step.
Figure 2 shows detection of Chlamydia pneumoniae DNA in CHD patients using assay methods as described herein. Lanes 1, 2 and 6 show results on CHD patients and lane 7 shows results on a healthy control.

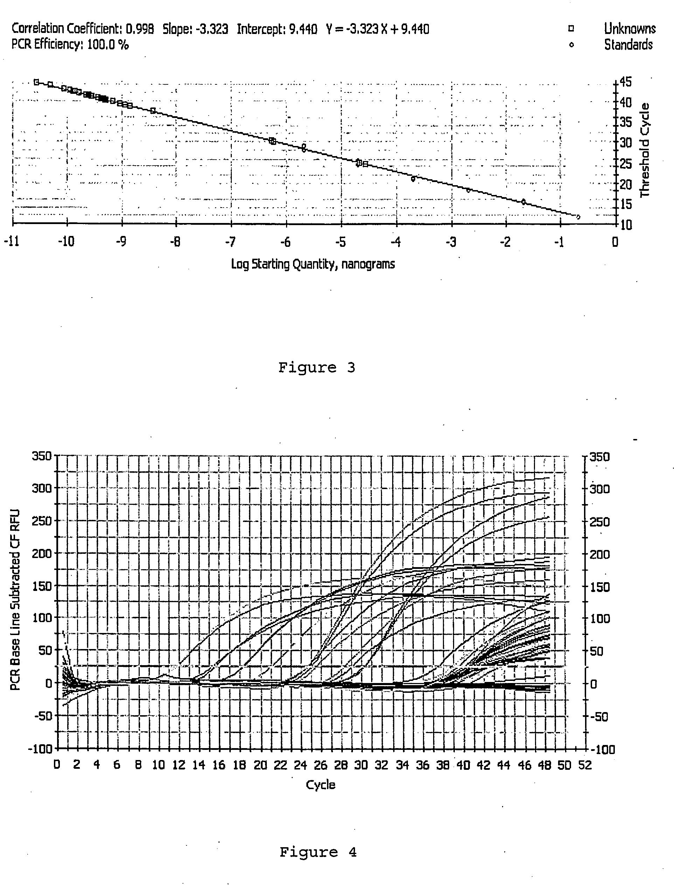

Figure 3 shows the real-time PCR standard curve graph using the fluorophore FAM-490.
Figure 4 shows the real-time PCR Amp/Cycle Graph for FAM-490.

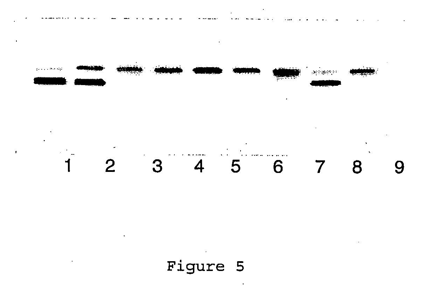

Figure 5 shows detection of Chlamydia pneumoniae DNA in samples using nested PCR. Lanes 6 and 7 show PCR negative samples P084 and P094 and lane 8 shows PCR positive sample P078.

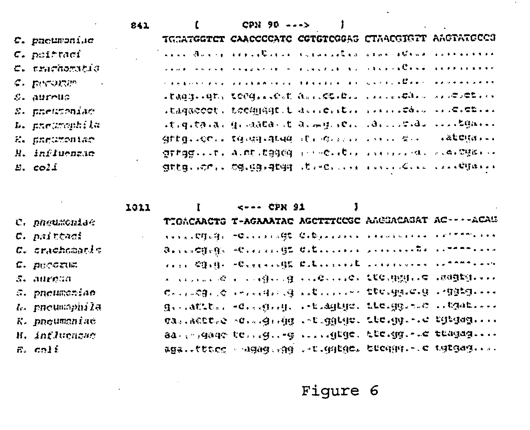

Figure 6 shows the alignment of the target sequences of primer set CPT 90/91 in the 16S rRNA gene of *C. pneumoniae* with the 16S rRNA genes of other pathogens. A dot indicates the same base, and a letter indicates a base different from that in *C. pneumoniae.* A dash indicates a deletion.
Figure 7 shows the level of Chlamydia antigen detected in patients using the present methods prior to and during anti-microbial therapy.

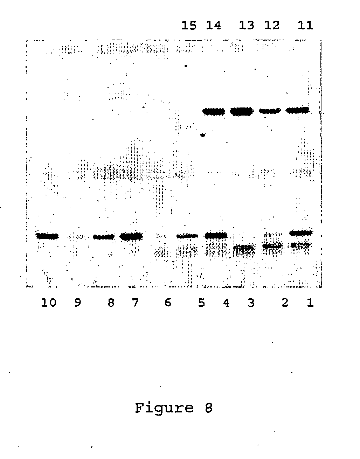

Figure 8 shows the detection of Chlamydia trachomatis DNA following concentration of *Chlamydia trachomatis* in human serum by using a column with low size pores as a bacteria retaining filter. The lanes are numbered as follows; 1, new protocol, concentration from 200µl of serum* 2, new protocol, concentration from 200µl of serum, 1:10*, 3, new protocol, concentration from 200µl of serum, 1:100*; 4, QIAGEN protocol 200µl of serum; 5, QIAGEN protocol 200µl of serum, 1:10; 6, QIAGEN protocol 200µl of serum, 1:100; 7, new protocol, concentration from 500µl* ;8, new protocol, concentration from 500µl, 1:10*; 9, new protocol, concentration from 500µl, 1:100*; 10, QIAGEN protocol 500µl of serum; 11, QIAGEN protocol 500µl of serum, 1:10; 12, QIAGEN protocol 500µl of serum, 1:100 13, 14 - positive control, 15 - negative control. * After concentration of the bacteria from serum samples, regardless of the volume used from 200µl up to 3,000µl, the standard QIAGEN protocol for treatment of samples of 200µl was used.

Table 1 shows a comparison of a PCR DNA detection assay based on blood serum as described herein with a conventional detection assay performed on whole blood. The patient group comprises individuals with CHD admitted for by-surgery and individuals with respiratory disease with a suspected *Chlamydia pneumoniae* involvement.

Table 2 shows a comparison of a PCR DNA detection assay based on blood serum as described herein with conventional detection assays performed on whole blood. The patient group comprises individuals with CHD/silent ischaemia. The control group comprises healthy individuals.

Table 3 shows the correlation of nucleic acid detection between conventional and real-time PCR.

Table 4 shows the detection of *Chlamydia trachomatis* in blood samples.

Table 5 shows detection of *Mycoplasma genitalium* in blood samples.

Table 6 shows detection of *Ureaplasma urealyticum (spp.)* in blood samples.

Table 7 shows a comparison of diagnostic value of serum DNA testing against swab diagnostic in the treatment of *C. trachomatis* genital infection.

Table 8 shows a comparison of diagnostic value of serum DNA testing against swab diagnostic in the diagnosis of acute *C.* trachomatis infection *(Vaginitis*/*cervicitis*/*urethritis)*

Table 9 shows a comparison of diagnostic value of serum DNA testing against swab diagnostic in the diagnosis of C. trachomatis infection in Reactive arthritis (Reiter's disease)

Table 10 shows a comparison of diagnostic value of serum DNA testing against swab diagnostic in the diagnosis of C. trachomatis infection in Chronic pelvic inflammatory disease

Table 11 shows a comparison of diagnostic value of serum DNA testing against swab diagnostic in the diagnosis of C. trachomatis infection in asymptomatic patients.

Table 12 shows an analysis of gene expression in C. trachomatis.

Table 13 shows the susceptibility of reference and persistent strains of C. trachomatis and C. pneumoniae to azithromycin

Table 14 shows the positive detection of Chlamydia pneumoniae DNA in blood by PCR relative to standard protocols, for control, CHD/Silent Ischaemia, and Peripheral Occlusive Disease patients.

Table 15 shows the detection of Chlamydia pneumoniae infection in blood for control and CHD patients.

### Experiments

### Material and Methods

### Samples

Venous blood samples were collected from three different clinical groups: from patients with CHD admitted for by-surgery; from patients with respiratory disease when *Chlamydia pneumoniae* involvement was suspected; and from clinically healthy individuals used as controls.

Venous blood samples were also collected from patients with and without signs of genital/urinary infection and from clinically healthy individuals used as controls.

After collection some samples were frozen and stored before testing at -20°C, some were kept at +4°C and some tested immediately after their collection.

Serum was separated from whole blood by centrifugation at 2,000g for 10 minutes.

### Concentration of cells using Protein A

Protein-A-agarose was co-incubated with the serum sample for 30-60 minute at 37°C.

The sorbent with the immobilised immunoglobulins (free and in immune complexes) was centrifuged for 10 min at 3,000-5,000g and the supernatant was discarded.

The sorbent pellet was then re-suspended in PBS and the centrifugation procedure was repeated. This washing step was repeated two more times.

After that, the re-suspended pellet was taken for DNA testing.

### Concentration of Cells by Filtration

Serum samples of 1,000 µl 200µl -2,000µl were loaded onto a QIAGEN silico-gel column and centrifuged for 5 min at 1,000g or for 1 min at 8,000 g. 200µl of QIAGEN buffer AL and 20µl of the protease was loaded onto the column and incubated for 10 min at 56°C. 200µl of ethanol was added and the column centrifuged for 1 min at 8,000 g. After that column manufacturer's protocol was followed.

Briefly, 500µl of AW1 buffer was added and centrifuged for 1 min at 8,000 g. 500µl of AW2 buffer was added and centrifuged for 3 min at 12,000 g. 100µl of AE buffer was added and the column incubated for 1 min and centrifuged for 1 min at 8,000 g. A 5-10 µl sample was then removed for PCR.

### PCR

DNA was extracted from 1 ml serum samples using the QIAamp DNA Blood Mini Kit (QIAGen) and resuspending in 100 µl of Buffer AE (QIAGen).

Extracted DNA was amplified by PCR using the following primers to the variable fragment of 16S rRNA gene, which are specific for *Chlamydia pneumoniae:* CPN90 -5'GGT CTC AAC CCC ATC CGT GTC GG 3'N CPN91 - 5' TGC GGA AAG CTG TAT TTC TAC AGT T 3' (Madico G. et al., J. Clin. Microbiol. 2000, 38:1085-1093). The size of the amplified fragment was 197 basepairs.

The amplification reactions were performed in 25 µl of PCR solution containing 10 mM Tris/HCl (pH 8.3), 50 mM KC1, 1.5 mM MgCl₂, 200 µM of each dNTP, 15 pmol of each primer, 1 U Taq-DNA-polymerase (Promega) and 5 µl of sample.

The following PCR programme was employed: 45 cycles of 45 s at 94°C, 45 s at 60°C, and 45 s at 72°C at the DNA thermocycler Perkin-Elmer Cetus.

The analytical sensitivity of the assay was 100 genome equivalent (GE) per PCR.

PCR products were visualized by ethidium bromide staining following electrophoretic separation in 1,2% agarose gel.

### Real-time PCR

Primers and probes were designed based on 16S rRNA gene of *Chlamydia pneumoniae,* using the design program 'Primer Express'

(Applied Biosystems) for "Taqman probes". Primers and probes used: Probe 553: 5'-CAAGTCCAGGTAAGGTCCTTCGCGTTGC-3', Probe 557: 5'-TCCAGGTAAGGTCCTTCGCGTTGCATCG-3', Primer CPN90: 5'-GGTCTCAACCCCATCCGTGTCGG-3', Primer CPN91: 5'-TGCGGAAAGCTGTATTTCTACAGTT-3'. The BLAST program was used to confirm the specificity of the selected primers against 16S rRNA genes of other species of Chlamydiaceae.

### Quantitative 16S rRNA-based real-time PCR.

The *C. pneumoniae*-specific sequences of the PCR primers and probe were selected from the 16S rRNA of *C. pneumoniae* (GenBank accession number AF131889.1 GI: 4545320 or AE009440.1 GI:33236960) with Primer Express Software (Applied Biosystems, Foster City, Calif.) and synthesized by Applied Biosystems. The PCR product generated was 197 bp; and the sequences of the primers and the TaqMan probe were as follows: forward primer CPN90, 5'-GGTCTCAACCCCATCCGTGTCGG-3'; reverse primer CPN91, 5'-TGCGGAAAGCTGTATTTCTACAGTT-3'; and TaqMan probe 557, 5'-TCCAGGTAAGGTCCTTCGCGTTGCATCG-3'. The TaqMan probe was fluorescence labelled at the 5' end with 6-carboxyfluoresceinas the reporter dye and at the 3' end with 6-carboxytetramethylrhodamine as the quencher. A search was performed with the BLAST program to check the specificities of the primers and probe.

The PCR product was detected as an increase in fluorescence during the PCR extension phase when the probe was cleaved by the 5' exonuclease activity of the *Taq* DNA polymerase. This cleavage interrupts the fluorescence resonance energy transfer and the reporter dye starts to fluoresce in proportion to the level of PCR product generated. The cycle threshold *(C_{T})* values, defined as the number of cycles at which the fluorescence of the reporter dye first exceeds the calculated background level, were automatically estimated by the instrument for each reaction. Standard graphs of *C_{T}* values obtained from serial dilutions of purified *C. pneumoniae* DNA. *C_{T}* values for unknown, clinical serum samples were plotted against the standard graphs for purified *C. pneumoniae* DNA.

DNA of cultured *C. pneumoniae* with known concentration was used as a standard in dilutions between 1 fg and 10 pg.

Real-time PCR was performed with the iCycler IQ system (BIO-RAD). 5 µl of extracted DNA was analyzed with the PCR mixture in a total volume of 50 µl. The PCR mixture consisted of 10 mM Tris (pH 8.3), 50 mM KCl, 1,5 mM MgCl₂, 200 µM of each dNTP, 5 U of Termostar *Taq* DNA polymerase (Sintol); 200 nM probe ; and 300 nM forward and reverse primers. The real-time PCR run was 10 min at 95°C, and 50 repeats of 1 min at 94°C and 1,5 min at 63°C. All samples were analyzed in triplicate. A sample was considered positive if three of three assay results were positive in the triplicate test and if the average value for the PCR run was greater than or equal to 1.0.

### Conventional C. pneumoniae Detection

Circulating PBMC were obtained by venipuncture into an 8-ml Vacutainer CPT cell preparation tube (BD Vacutainer Systems, Franklin Lakes, N.J.). CPT tubes contain a blood separation medium composed of a thixotropic polyester gel and a density gradient liquid solution. Briefly, CPT tubes were centrifuged in a Beckman GPR centrifuge at 1,500 × *g* for 30 min and refrigerated. After transport to the research laboratory, the specimens were mixed by inversion and recentrifuged, and the mononuclear cell layer or 1 ml of plasma directly above the gel was aspirated and frozen at -70°C. In batches, mononuclear cell preparations were thawed and 200-µl aliquots were extracted using QIAamp DNA minikits (Qiagen, Mississauga, Ontario, Canada) into 100 µl of elution buffer.

### Nested PCR detection of C. pneumoniae

A nested PCR was used which targeted the variable domain IV (VDIV) region of the *ompA* gene (outer primers Cpn5P [5' CCA ATA TGC ACA GTC CAA ACC TAA AA 3'] and Cpn3P [5' CTA GAT TTA AAC TTG TTG ATC TGA CAG 3']; nested primers -Cpn5N [5' CTC TGT AAA CAA ACC GGG C 3'] and Cpn3N [5' GAT CTG ACA GGA AAC AAT TTG CAT 3']).

The amplification reactions were performed in 25 µl of PCR solution containing 10 mM Tris/HCl (pH 8.3), 50 mM KCl, 1.5 mM MgCl₂, 200 µM of each dNTP, 15 pmol of each primer, 1 U Taq-DNA-polymerase (Promega) and 5 µl of sample.

Cycling conditions consisted of an initial denaturation for 5 min at 95°C, followed by 45 cycles of denaturation at 95°C for 30 sec, annealing at 60°C for 30 sec, and extension for 30sec at 72°C. For the second round of PCR, 2 µl of the first-round,product was mixed with 25 µl of the above amplification mixture, using primers Cpn5N and Cpn3N, and amplified under the same cycling conditions.

PCR products were visualized by ethidium bromide staining following electrophoretic separation in 1.2 % agarose gel.

### Detection of C. trachomatis

A specific DNA fragment of the cryptic plasmid of *Chlamydia trachomatis* was used for detection of this pathogen. The following primer set, generating a 210- bp fragment, was:
CP 24 - 5' GGGATTCCTGTAACAACAAGTCAGG 3'
CP 27 - 5' CCTCTTCCCCAGAACAATAAGAAC 3'.

PCR was performed in 25 µl of PCR solution containing 10 mM Tris/HCl (pH 8.3), 50 mM KCl, 1.5 mM MgCl₂, 200 µM of each dNTP, 15 pmol of each primer, 1 U Taq-DNA-polymerase (Promega) and 5 µl of sample.

Forty-five cycles of amplification were performed with a PCR Thermocycler Perkin Elmer. Each cycle consisted of denaturation step at 94°C for 45 sec, primer annealing at 55°C for 45 sec, primer extension at 72°C for 45 sec. Amplified product (10 µl*)* was visualised by electrophoresis in a 1.5% agarose gel and stained with ethidiumbromide.

### Detection of M. genitalium

PCR specific for the *Mycoplasma genitalium* targeted the region of 140 kDa adhesion protein gene, MgPa (Jensen JS et al J. Clin. Microbiol. 1991 Jan; 29(1):46-50).

The following primer set, generating a 281- bp fragment, was used:
MgPa-1 - 5' AGTTGATGAAACCTTAACCCCTTGG 3'
MgPa-3 - 5' CCGTTGAGGGGTTTTCCATTTTTGC 3'.

The amplification reactions were performed in 25 µl of PCR solution containing 10 mM Tris/HCl (pH 8.3), 50 mM KCl, 1.5 mM MgCl₂, 200 µM of each dNTP, 15 pmol of each primer, 1 U Taq-DNA-polymerase (Promega) and 5 µl of sample.

Forty-five cycles of amplification were performed with a PCR Thermocycler Perkin Elmer. Each cycle consisted of denaturation step at 94°C for 45 sec, primer annealing at 55°C for 45 sec , primer extension at 72°C for 45 sec.

Amplified product (10 µl) was visualised by electrophoresis in a 1.5% agarose gel and stained with ethidium bromide.

### Detection of Ureaplasma urealyticum

PCR was performed with primers specific for highly conserved regions in the urease gene of *Ureaplasma* spp. (Blanchard A et al Clin Infect Dis. 1993 Aug;17 Suppl 1:S148-53.)

The following primer set, generating a 429- bp fragment, was used:
U1 - 5' GATGGTAAGTTAGTTGCTGAC 3'
U2 - 5' ACGACGTCCATAAGCAACT 3'.

The amplification reactions were performed in 25 µl of PCR solution containing 10 mM Tris/HCl (pH 8.3), 50 mM KCl, 1.5 mM MgCl₂, 200 µM of each dNTP, 15 pmol of each primer, 1 U Taq-DNA-polymerase (Promega) and 5 µl of sample.

Forty-five cycles of amplification were performed with a PCR Thermocycler (Perkin Elmer). Each cycle consisted of denaturation step at 94°C for 45 sec, primer annealing at 55°C for 45 sec, primer extension at 72°C for 45 sec.

Amplified product (10 µl) was visualised by electrophoresis in a 1.5% agarose gel and stained with ethidium bromide.

### Cell Culture

For Chlamydia trachomatis isolation McCoy cells were used and for Chlamydia pneumoniae - HL cells.

Cells of each cell line were seeded into 24-well cell culture plates (approximately 2 x 105 cells per well) with cover-slips and incubated at 37°C for 24 h to achieve monolayer confluence.

1 ml of serum was centrifuged at 14,000 x g for 1 h at 4°C to sediment bacterial cells. Supernatant was discarded and pellet was suspended in 0,5 ml of GM (growth medium: RPMI, 5% [vol/vol] fetal calf serum, 2.0 mM L-glutamine, 8.8% [vol/vol] glucose, 4.0 µg of gentamicin per ml and 5.0 of amfotherycin B/ml). The cell monolayers were inoculated with the suspensions and centrifuged at 2,500 x g for an hour at 20°C. The infected cells were incubated for 2 h at 37°C; after that, the medium was removed and replaced by GM with cycloheximide (1.0 µg /ml). The cells were incubated at 37°C in 4% CO2 for 72 h.

The cells on the cover-slips were fixed with acetone and stained with a fluorescein isothiocyanate (FITC)-labeled Chlamydia genus-specific (anti-LPS) and Chlamydia trachomatis-specific (anti-MOMP) antibodies. Chlamydia inclusions were detected by fluorescent microscopy.

The isolation of a mixture of Chlamydia and cellular DNAs was performed by using QIAGEN Mini Kit according to the manufacture's instructions. DNA was eluted by 100 µl of TE buffer.

### PCR

The commercially available PCR kits for Chlamydia trachomatis and Chlamydia pneumoniae were used.

### RNA isolation

Total RNA was isolated by SV Total RNA Isolation System (Promega) according to the manufacture's instructions. RNA was treated by RNase free DNase I enzyme and eluted from Spin Basket by 100 µl of Nuclease-Free Water. RNA samples were tested for the genomic DNA contamination.

### RT-PCR

Gene expression of 16 S rRNA, HSP60, MOMP and other outer membrane proteins of C.trachomatis were analyzed by RT-PCR. The nucleotide sequences of the used for RT-PCR primers are the following:
16 s rRNA
   CTR 70 5' -GGC GTA TTT GGG CAT CCG AGT AAC G 3'
   CTR 71 5' -TCA AAT CCA GCG GGT ATT AAC CGC CT 3'
   MOMP -L 5' CGTTCGTTGCAGACTTACCA
   MOMP - R 5' GTTCCTCGCATACCGAATGT
   OMCB-L 5'- CTGCAACAGTATGCGCTTGT
   OMCB-R 5' -CACGCTGTCCAGAAGAATGA
   OMCA-L 5' - GTTGCTTCGAAGATCCATGC
   OMCA-R 5' - GGGCCATGTTTAGCATCTTG
   PMPA-L 5' - GCATTTAGCGGCAATACCAT
   PMPA-R 5'- TGACAATGCCATGACAGGAT
   PMPB-L 5'-GAAGGCGGTGCTATCTTCTCTC
   PMPB -R 5'- TCGCTTGCTGTTTGAGCTTTAG
   PMPC-L 5'- CACCTACGACAACACCAACG
   PMPC-R 5'- GGAGCAATATCACCCGTCAG .
   PMPD-L 5'- GTTAGACCAAATTCGAGATC
   PMPD-R 5'- AAGATTCTCCGTCACGAGGA
   PMPE-L 5'-CTAACTGCTATCTCGATAACC
   PMPE-R 5'- TCACGAATCTCCACGGTAGG
HSP60
   GroEL-L 5' - TCTGCGAACGAAGGATATGA
   GroEL -R 5' - ATAGTCCATTCCTGCGCCAGG

### Antibiotic susceptibility test

McCoy and HL cells were seeded and inoculated with serum samples as described above but after centrifugation at 2,500 x g for an hour and incubation for 2 h at 37°C the infected cells were overlaid with the growth medium containing serial twofold dilutions of the antibiotic (Azithromycin, Pliva) at concentration range from 0.05 to 0.8 µg/ml and incubated at 37°C in 4% CO₂ for 72 h. Chlamydia trachomatis L-2 and Chlamydia pneumoniae Kajaani 6 strains were used as reference strains. The MIC was defined as the lowest concentration at which no inclusions were detected.

### Results

PCR detection of *C*. *pneumoniae* DNA in the serum of 60 samples from all patient groups was performed and compared to the rate of DNA detection by a conventional phenol extraction PCR-based assay in samples of whole blood from the same patients. *C*. *pneumoniae* DNA was amplified and detected in 38% of the patient pool (23 out of 60 patients) using the PCR DNA detection assay based on blood serum described herein. In contrast, the conventional PCR DNA detection assay based on DNA isolated from circulating PBMC detected *C*. *pneumoniae* DNA only in 1.7% of the same patient pool (1 out of 60) (see table 1). The new technique increased the sensitivity of detecting a bacterial DNA infection in patients by 22 fold.

PCR detection of *C*. *pneumoniae* DNA in the serum of patients with CHD/Silent Ischaemia was performed in comparison to a conventional PCR-based assay using DNA isolated from circulating PBMC and in comparison to a control group of healthy individuals.

The conventional PCR DNA detection assay based on whole blood detected *C*. *pneumoniae* DNA only in 3.9% of CHD/Silent Ischaemia patients (4 out of 102 patients) (see table 2). Such a low level of DNA recovery does not allow PCR to be a meaningful diagnostic method for the detection of *C*. *pneumoniae* infection in patients with CDH. In contrast, when using the described PCR DNA detection assay base on blood serum, *C*. *pneumoniae* DNA was amplified and detected in 58% of CHD/Silent Ischaemia patients (14 out of 24 patients). The new technique increased the sensitivity of detecting a bacterial DNA infection in patients by 15 fold.

In the control group of healthy individuals the same technique detected *C*. *pneumoniae* DNA in only 4.8% of cases (1 out of 22 control individuals).

Real-time PCR detection of *C*. *pneumoniae* DNA in the serum of patients with CHD/Silent Ischaemia was performed in comparison to a conventional PCR-based assay on the same serum samples.

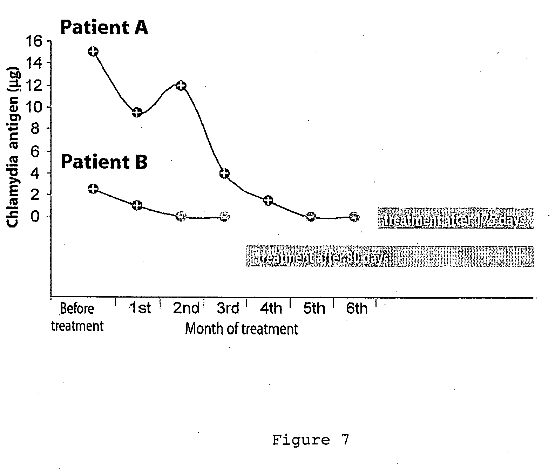

Whereas the conventional PCR method could only determine the presence of Chlamydia pneumonia in a qualitative manner, the real-time PCR based method allowed the quantification of the DNA present in the samples. The amount varied from 20 -180 *Chlamydia pneumoniae* genome equivalents in 1 ml of blood, indicating varying bacterial load in the infected individuals (table 3). *Chlamydia pneumoniae* DNA was detected in blood serum samples using nested PCR on the *ompA* gene (figure 7).

The detection of Chlamydia trachomatis in serum was compared to detection in urea and swabs in patients with acute genital infection and chronic inflammation of reproductive system and/or infertility problems. The results are shown in Table 4. Chlamydia trachomatis was detected by PCR or MIF in the serum of the five patients with acute genital infection who tested negative using urea or swabs. *Chlamydia trachomatis* was detected by PCR or MIF in the serum of six patients with chronic inflammation of reproductive system and/or infertility problems who tested negative using urea or swabs.

Concentration of *C trachomatis* in serum samples was also performed in the absence of protein A, using a silico-gel column (Qiagen) with low size pores as a bacteria retaining filter. *C trachomatis* cells were observed to be successfully concentrated from serum samples using these columns (Figure 8).

The detection of Mycoplasma genitalium in serum was compared to detection in urea and swabs in patients with acute genital and/or urinary infection and chronic inflammation of reproductive system and/or infertility problems. The results are shown in Table 5. Mycoplasma genitalium was detected by PCR or MIF in the serum of two patients with acute genital and/or urinary infection who tested negative using urea or swabs. Mycoplasma genitalium was detected by PCR or MIF in the serum of three patients with chronic inflammation of reproductive system and/or infertility problems who tested negative using urea or swabs.

The detection of *Ureaplasma urealyticum* in serum was compared to detection in urea and swabs in patients. The results are shown in Table 6. *Ureaplasma urealyticum* was detected by PCR or MIF in the serum of three patients who also tested positive using urea or swabs.

The effect of anti-microbial treatment on patients diagnosed as having *Chlamydia trachomatis* infection was investigated. Two patients diagnosed as having *Chlamydia trachomatis* infection were treated with 400 mg twice daily of Avelox (moxifoxacin hydrochloride) and the presence of *Chlamydia trachomatis* infection determined by both conventional swab assays and the blood tests described herein. The results are shown in table 7. In both cases, the blood tests described herein continued to detect the presence of *Chlamydia trachomatis* when convention tests were negative.

This provides indication that the blood tests described herein reduce a number of false negative cases in the diagnostics of *Chlamydia trachomatis.* Furthermore the efficacy of anti-microbial treatment is shown to be increased by the use of the present blood tests, because the chances of complete eradication of the infection are increased. This may also be useful in preventing the development of chronic persistent forms of the infection.

The Serum DNA test was compared to standard DNA Swab tests for Chlamydia trachomatis for patients with acute

Vaginitis/cervicitis/urethritis and the results are shown in Table 8. In a total of 13 patients, 11 (85%) tested positive by swab test and 12 (92%) tested positive by blood test.

Table 9 shows the results of a clinical validation of Serum DNA test in comparison with DNA Swab test for Chlamydia trachomatis in reactive arthritis (Reiter's disease). In a total of 15 patients, 8 (53%) tested positive by swab test and 12 (80%) tested positive by blood test.

Table 10 shows the results of a clinical validation of Serum DNA test in comparison with DNA Swab test for Chlamydia trachomatis in chronic pelvic inflammatory disease (Vaginitis, cervicitis, endometriosis, urethritis, epididymitis and prostatitis). In a total of 215 patients, 17 (8%) tested positive by swab test and 57 (26.5%) tested positive by blood test.

Table 11 shows the results of a clinical validation of Serum DNA test in comparison with DNA Swab test for Chlamydia trachomatis in asymptomatic patients. In a total of 124 patients, 3 (2.4%) tested positive by swab test and 24 (19%) tested positive by blood test.

The present blood tests were found to reduce false negative diagnosis for Chlamydia trachomatis in Reactive arthritis patients by 50%, chronic pelvic inflammatory disease patients by 335%, and in patients with asymptomatic infection by 800%.

It was noted that a random check of donor blood samples by the serum test showed that 2 out of 11, or 18%, were positive for Chlamydia trachomatis DNA.

Analysis of gene expression is shown in Table 12.

Immunochemical detection of Chlamydia trachomatis serum isolates by staining with the anti-LPS (genus-specific) and the anti-MOMP (species-specific) monoclonal antibodies showed decreasing of LPS and MOMP in the cell membrane. LPS of the serum isolates was significantly restored in 1st or 2nd passages on cell culture. After 3 passages, MOMP was detected at the same reduced level.

Azithromycin susceptibility is shown in Table 13.

The results of the new PCR test for Chlamydia pneumoniae in the blood of patients with Coronary Heart Disease and Peripheral Occlusive Disease are shown in Table 14.

Results of direct and cell culture PCR tests in diagnosis of Chlamydia pneumoniae infection in the blood of control and CHD patients is shown in Table 15.

The data set out above shows that the present methods are useful in the detection of infection with an ntracellular pathogens such as *C*. *trachomatis, C. pneumoniae, Mycoplasma genitalium,* or *Ureaplasma urealyticum.* It is important to note that there are no other currently available blood tests for these four infections which can be used for the blood bank screening.

The methods and kits described herein may be useful in treating and prevent spreading of these persistent blood-borne infections and associated disease conditions.

### References

1. Fabricant CG et al, J Exp Med 1978, 148(1):335-340.
2. Fong IW: CMAJ 2000, 163 (1) :49-56
3. Mattila KJ et al, Clin Infect Dis 1998, 26(3) :719-734
4. Campbell LA et al, Emerg Infect Dis 1998, 4(4):571-579
5. Saikku P et al, Lancet 1988, 2(8618):983-986
6. Danesh J et al, Lancet 1997, 350(9075):430-436
7. Danesh J et al, BMJ 2000, 321(7255):208-213
8. Boman J et al, Clin Microbiol Rev 2002, 15(1):1-20
9. Shor A et al, J Clin Pathol 1998, 51(11):812-817
10. Campbell LA et al, J Infect Dis 1995, 172(2):585-588
11. Dechend R et al, Circulation 1999, 100(13):1369-1373
12. Coombes BK et al, Infect Immun 1999, 67(6):2909-2915
13. Jackson LA et al, Am J Pathol 1997, 150(5):1785-1790
14. Apfalter P et al, J Clin Microbiol 2001, 39(2) :519-524
15. Blasi F et al, J Infect Dis 2000, 181(Suppl 3):S444-S446
16. Ramirez JA, Ann Intern Med 1996, 125(12):979-982
17. Jackson LA et al, J Infect Dis 1997, 176(1) :292-295
18. Apfalter P et al, Eur J Clin Microbiol Infect Dis 2000, 19(4):305-308
19. Karlsson L et al, Eur J Vasc Endovasc Surg 2000, 19(6):630-635
20. Bartels C et al, Circulation 2000, 101(2):137-141
21. Berger M et al, J Lab Clin Med 2000, 136(3):194-200
22. Boman J et al, J Infect Dis 1998, 178(1):274-277
23. Freidank HM et al, Eur J Clin Microbiol Infect Dis 2002, 21(1):60-62
24. Kaul R et al, Circulation 2000, 102(19):2341-2346
25. Maraha B et al, Eur J Clin Microbiol Infect Dis 2001, 20(2):111-116
26. Sessa R et al, Atherosclerosis 2001, 159(2):521-525
27. Smieja M et al, J Clin Microbiol 2001, 39(2):596-600
28. Wong YK et al, J Am Coll Cardiol 1999, 34(5):1435-1439
29. Maass M et al, J Infect Dis 2000, 181(Suppl 3):S449-S451
30. Blasi F et al, J Infect Dis 1999, 180(6):2074-2076
31. Taylor-Robinson D et al, J Am Coll Cardiol 2000, 36(2):657-658
32. Smieja M et al, BMC Infect Dis 2002, 2(1) :12
33. Iliescu EA et al, Perit Dial Int 2000, 20(6):722-726
34. Bodetti TJ et al, Infect Immun 2000, 68(5):2744-2747
35. Rassu M et al, Med Microbiol Immunol 2001, 190:139-144
36. Haranaga S et al, Transfusion 2001, 41(9):1114-1119
37. Hahn DL et al, Arterioscler Thromb 1992, 12(8):945-947
38. Von Hertzen et al, J Med Microbiol 1998, 47(5):441-446
39. Tong CY et al, J Clin Pathol 1993, 46(4):313-317
40. Egger M et al, BMJ 1997, 315(7109):629-634
41. Boman J et al, J Clin Microbiol 1999, 37(12):3791-3799
42. Boman J et al, J Infect Dis 2000, 181(Suppl 3):S452-S454
43. Smieja M et al, J Clin Microbiol 2001, 39(5):1796-1801
44. Chernesky M et al, J Clin Microbiol 2002, 40(7):2357-2362
45. Mahony JB et al, J Clin Microbiol 2000, 38(7):2622-2627
46. Gieffers J et al, Circulation 2001, 103(3):351-356
47. Smieja et al, BMC Infectious Diseases 2002, 2:21
48. Madico G. et al, J Clin Microbiol 2000, 38:1085-1093
49. Arya M. et al, Expert Rev Mol Diagn 2005, 5:209-219
50. Klein D, Trends Mol Med 2002, 8:257-260
51. Ausuble et al, Current Protocols in Molecular Biology, John Wiley & Sons, 1992
52. Russell et al, Molecular Cloning: a Laboratory Manual, 3rd edition, 2001, Cold Spring Harbor Laboratory Press

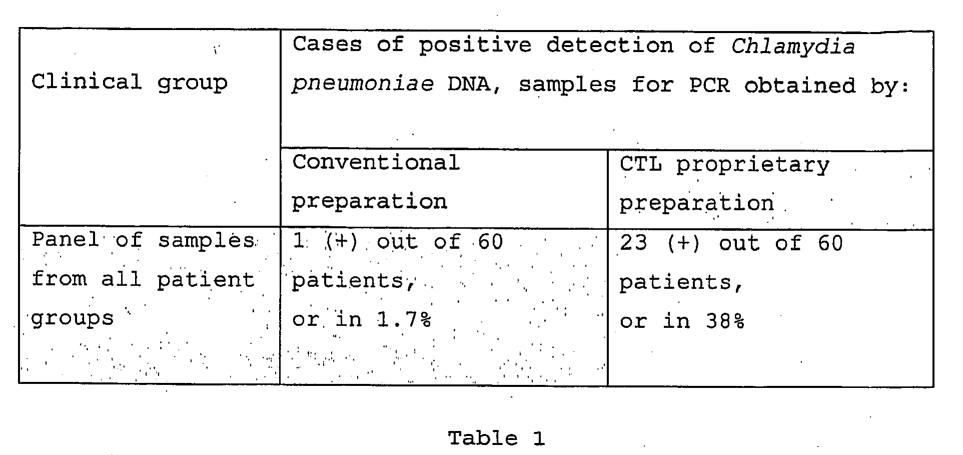

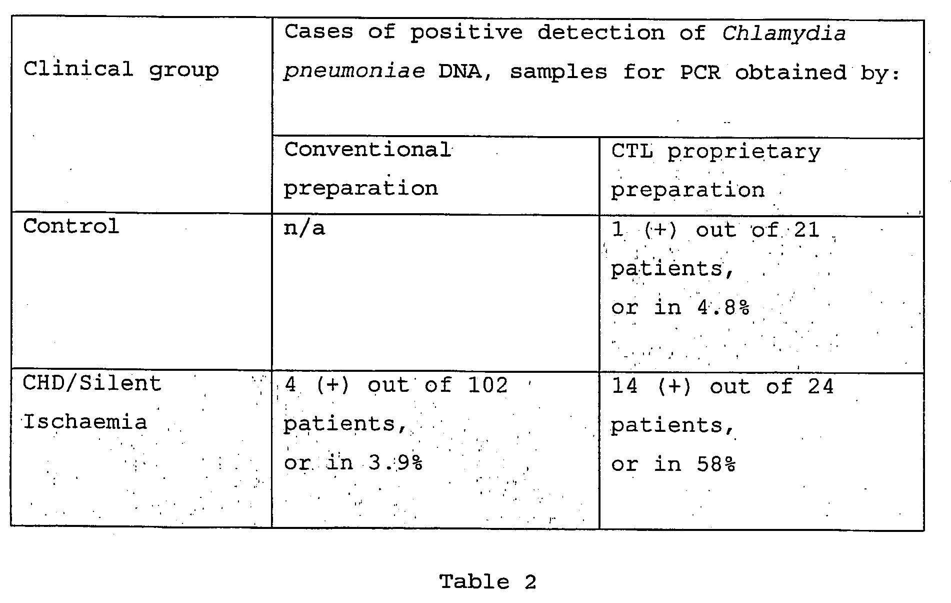

**Table 3**

| Patients | Conventional PCR, detection of Chlamydia pneumoniae DNA | Real-Time PCR, number of Chlamydia pneumoniae genome equivalents in 1 ml of blood |
|---|---|---|
| 33 | + | 0 |
| Kolk | + | 90 |
| P151 | - | 0 |
| P153 | + | 40 |
| P 026 | - | 0 |
| P 042 | + | 28 |
| P 044 | + | 22 |
| P 046 | + | 45 |
| P 052 | + | 20 |
| P 059 | - | 0 |
| P 065 | + | 80 |
| P 071 | + | 180 |
| P 078 | + | 100 |
| P 083 | - | 0 |
| P 084 | - | 0 |
| P 087 | - | 0 |
| P 094 | - | 0 |
| P 095 | - | 110 |
| P 096 | - | 0 |
| P 097 | - | 0 |

**Table 4**

| Patients | Swab and urea samples, DNA by PCR | Serum | |
|---|---|---|---|
| | | DNA by PCR | IgG antibodies by MIF |
| Acute genital infection | | | |
| 1 | + | + | - |
| 2 | - | + | - |
| 3 | - | + | - |
| 4 | - | - | + |
| 5 | + | - | - |
| 6 | + | + | - |
| 7 | - | + | - |
| 8 | - | - | + |

| Chronic inflammation of reproductive system and/or infertility problems | | | |
|---|---|---|---|
| 9 | - | - | + |
| 10 | - | + | - |
| 11 | - | - | - |
| 12 | - | + | - |
| 13 | - | - | - |
| 14 | - | - | + |
| 15 | - | + | - |
| 16 | - | + | - |

**Table 5**

| Patients | DNA by PCR in: | |
|---|---|---|
| | Swab and urea samples | Serum |
| Acute genital and/or urinary infection | | |
| 1 | + | + |
| 2 | - | - |
| 3 | - | - |
| 4 | - | + |
| 5 | - | - |
| 6 | - | + |

| Chronic inflammation of reproductive or urinary systems | | |
|---|---|---|
| 7 | - | - |
| 8 | - | + |
| 9 | - | - |
| 10 | - | + |
| 11 | - | + |
| 12 | - | - |

**Table 6**

| Patients | DNA detection by PCR | |
|---|---|---|
| | Swab and urea samples | Serum |
| 1 | + | + |
| 2 | + | + |
| 3 | + | + |
| 4 | | - |
| 5 | + | - |
| 6 | - | - |
| 7 | - | - |
| 8 | + | - |
| 9 | - | - |
| 10 | - | - |
| 11 | - | - |
| 12 | - | - |
| 13 | + | - |
| 14 | - | - |

**Table 7**

| Patients | Patient S | | Patient V | |
|---|---|---|---|---|
| Tests | PCR diagnostic of Chlamydia trachomatis DNA in: | | | |
| | swab | blood | swab | blood |
| Before treatment | + | + | - | + |
| Diagnosis | combined genital and blood borne infection | | blood borne infection | |
| Anti-microbial treatment | - | + | - | + |
| • 14-15^{th} day | - | + | - | - |
| • 30-31^{st} day | - | - | | |
| • 48-50^{th} day | | | end of the treatment | |
| | end of the treatment | | | |

**Table 8**

| | | DNA in Swab, PCR | |
|---|---|---|---|
| | | negative | positive |
| DNA in Blood, PCR | negative | 1 (7.7%) | 0 |
| | positive | 1 (7.7%) | 11 (85%) |

**Table 9**

| | | DNA in Swab, PCR | |
|---|---|---|---|
| | | negative | positive |
| DNA in Blood, PCR | negative | 3 (20%) | 0 |
| | positive | 4 (27%) | 8 (53%) |

**Table 10**

| | | DNA in Swab, PCR | |
|---|---|---|---|
| | | negative | positive |
| DNA in Blood, PCR | negative | 158 (73.5%) | 0 |
| | positive | 40 (19%) | 17 (8%) |

**Table 11**

| | | DNA in Swab, PCR | |
|---|---|---|---|
| | | negative | positive |
| DNA in Blood, PCR | negative | 100 (80%) | 0 |
| | positive | 21 (17%) | 3 (2.4%) |

**Table 12**

| | Gene expression | | |
|---|---|---|---|
| Chlamydia trachomatis proteins | L-2 strain (reference) | Persistent forms of the bacteria | |
| | | Isolated from patient serum | Artificially obtained in vitro* |
| 16S rRNA | + | + | + |
| HSP60 | + | +++ | +++ |
| MOMP | +++ | + | +/- |
| Omp2 (60 kDa) | + | - | - |
| Omp3 (9 kDa) | + | - | - |
| PmpA | + | + | n/a |
| PmpB | + | + | n/a |
| PmpC | + | + | n/a |
| PmpD | + | - | +/- |
| PmpE | + | + | +/- |

| | | | |
|---|---|---|---|
| +/- down regulation *by nutrients deprivation in cell culture | | | |

**Table 13**

| Azithromycin | Chlamydia trachomatis | | | Chlamydia pneumoniae | | |
|---|---|---|---|---|---|---|
| | Reference strain L-2 | Isolated from serum of: | | Reference strain Kajaani 6 | Isolated from serum of: | |
| | | Patient A | Patient B | | Patient C | Patient D |
| Range of anti-microbial concentration, in µg/ml | > 0.1 | > 0.4 | > 0.4 | > 0.05 | > 0.4 | > 0.4 |

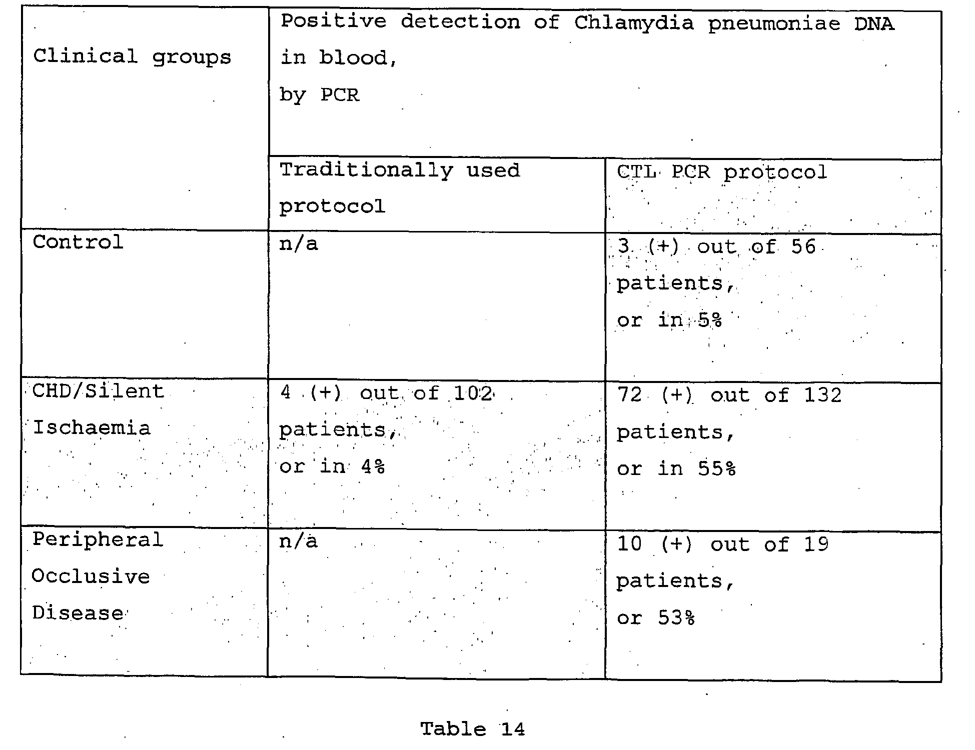

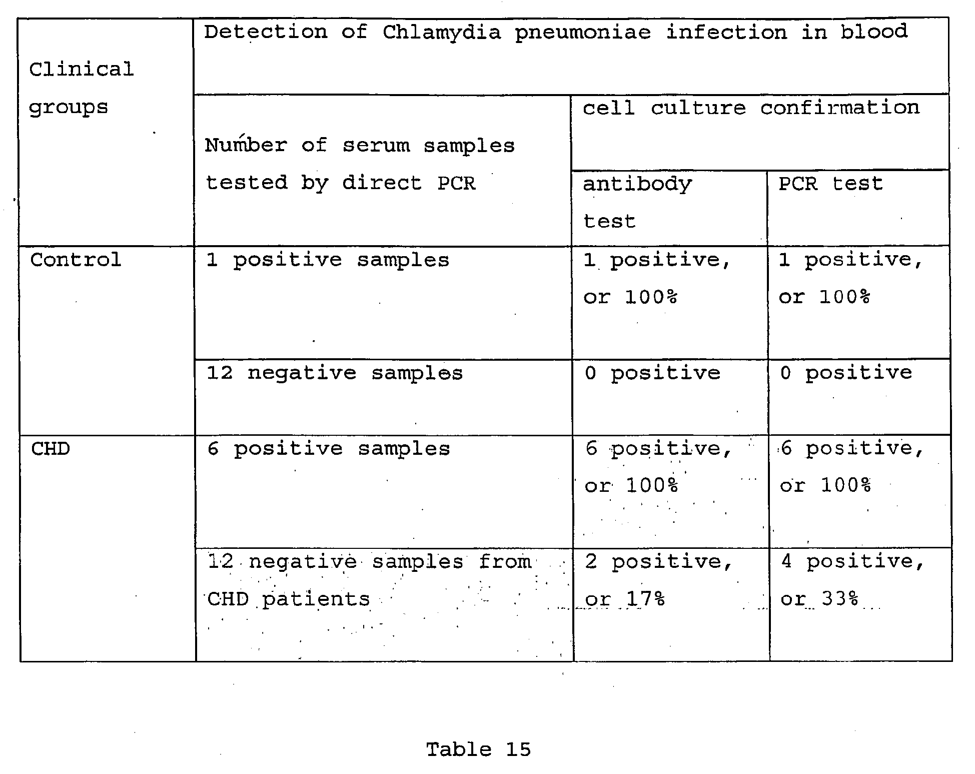

## Claims

1. A method for assessing an individual for infection with a persistent form of an obligate intracellular pathogenic bacterium comprising:
contacting an acellular blood sample from the individual with an specific binding member to isolate and/or purify a fraction of said sample,
wherein the specific binding member binds to a complex comprising one or more host plasma/serum molecules selected from the group consisting of antibodies, serum lipoproteins or lipopolysaccaride binding proteins, and the isolated and/or purified fraction comprises one or more said complexes, and
wherein the specific binding member is selected from the group consisting of protein A and antibodies which bind to immunoglobulin, serum lipoproteins or lipopolysaccaride binding proteins, and;
determining the presence of nucleic acid from the pathogenic bacterium in said fraction.

2. A method according to claim 1 comprising;
concentrating and/or extracting DNA from the isolated and/or purified fraction, and;
determining the presence of pathogen nucleic acid in the concentrated and/or extracted nucleic acid.

3. A method according to claim 1 or claim 2 comprising removing host blood cells from a blood sample to produce the acellular blood sample.

4. A method according to any one of claims 1 to 3 wherein the one or more plasma/serum molecules are antibodies and the complex is an immunocomplex.

5. A method according to claim 4 wherein the specific binding member binds to immunoglobulin.

6. A method according to claim 5 wherein the specific binding member is protein A.

7. A method according to any one of claims 1 to 3 wherein the plasma/serum molecules are serum lipoproteins or lipopolysaccaride binding proteins.

8. A method according to claim 7 wherein the specific binding member binds to said serum lipoproteins or lipopolysaccaride binding proteins.

9. A method according to any one of the preceding claims wherein the presence of the pathogen nucleic acid in said fraction is indicative of the presence of the pathogen infection in the individual.

10. A method according to claim 9 wherein the presence of the pathogen nucleic acid in said fraction is indicative of the presence of a disorder associated with pathogen infection.

11. A method according to claim 10 wherein the presence of a *C*. *pneumoniae* nucleic acid is indicative of the presence of an atherosclerotic condition in the individual.

12. A method according to claim 10 wherein the presence of a *C*. *trachomatis* nucleic acid is indicative of the presence of reproductive dysfunction of the individual.

13. A method according to claim 10 wherein the presence of a *C*. *trachomatis* nucleic acid is indicative of the presence of an infection selected from the group consisting of vaginitis, cervicitis and urethritis.

14. A method according to claim 10 wherein the presence of a *C. trachomatis* nucleic acid is indicative of the presence of Reactive arthritis (Reiter's disease).

15. A method according to claim 10 wherein the presence of a *C. trachomatis* nucleic acid is indicative of the presence of chronic pelvic inflammatory disease

16. A method according to claim 10 wherein the presence of a *C. trachomatis* nucleic acid is indicative of the presence of asymptomatic infection.

17. A method according to claim 10 wherein the presence of a *Mycoplasmataceae* nucleic acid is indicative of the presence of respiratory disorder, pelvic inflammatory disease or reproductive dysfunction in the individual.

18. A method according to any one of the preceding claims wherein the presence of pathogen nucleic acid in the isolated and/or purified fraction is determined by amplification with pathogen specific primers,
the presence of amplification products being indicative of the presence of pathogen nucleic acid.

19. A method according to claim 18 wherein the presence of pathogen nucleic acid in the sample is determined by PCR using pathogen specific primers.

20. A method according to any one of the preceding claims wherein the pathogen is a Chlamydia spp.

21. A method according to claim 20 wherein the Chlamydia spp is *C*. *pneumoniae* or *C. trachomatis.*

22. A method according to claim 20 or claim 21 wherein the presence of the Chlamydia cell is determined by determining the presence of Chlamydia nucleic acid using Chlamydia specific primers.

23. A method according to claim 22 wherein the Chlamydia is *Chlamydia pneumoniae.*

24. A method according to claim 23 wherein the Chlamydia specific primers amplify all or part of the 16S RNA gene of *Chlamydia pneumoniae.*

25. A method according to claim 24 wherein the primers are selected from the group consisting of:
5' GGT CTC AAC CCC ATC T 3'
5'-GGT CTC AAC CCC ATC CGT GTC GG-3'
5' TGC GGA AAG CTG TAT TTC TAC AGT T 3'
5'-CAAGTCCAGGTAAGGTCCTTCGCGTTGC-3' and,
5'-TCCAGGTAAGGTCCTTCGCGTTGCATCG-3'

26. A method according to claim 23 wherein the Chlamydia specific primers amplify all or part of the OmpA gene of *Chlamydia pneumoniae.*

27. A method according to claim 26 wherein the primers are selected from the group consisting of:
5' CCA ATA TGC ACA GTC CAA ACC TAA AA 3'
5' CTA GAT TTA AAC TTG TTG ATC TGA CAG 3'
5' CTC TGT AAA CAA ACC GGG C 3'
5' GAT CTG ACA GGA AAC AAT TTG CAT 3'

28. A method according to claim 22 wherein the pathogen is *Chlamydia trachomatis.*

29. A method according to claim 28 wherein the *Chlamydia trachomatis* specific primers amplify all or part of the *C*. *trachomatis* cryptic plasmid

30. A method according to claim 29 wherein the primers are:
CP 24 - 5' GGGATTCCTGTAACAACAAGTCAGG 3'
CP 27 - 5' CCTCTTCCCCAGAACAATAAGAAC 3'.

31. A method according to any one of claims 1 to 19 wherein the pathogen is a *Mycoplasmataceae* species.

32. A method according to claim 31 wherein the pathogen is a mycoplasma.

33. A method according to claim 32 wherein the mycoplasma is *M.genitalium.*

34. A method according to claim 33 wherein the presence of the *Mycoplasma genitalium* is determined by determining the presence of *Mycoplasma genitalium* nucleic acid using *Mycoplasma genitalium* specific primers

35. A method according to claim 34 wherein the *Mycoplasma genitalium* specific primers amplify all or part of the MgPa gene.

36. A method according to claim 35 wherein the primers are:
MgPa-1 - 5' AGTTGATGAAACCTTAACCCCTTGG 3'
MgPa-3 - 5' CCGTTGAGGGGTTTTCCATTTTTGC 3'

37. A method according to claim 31 wherein the pathogen is a ureaplasma.

38. A method according to claim 37 wherein the ureaplasma is *U*. *urealyticum.*

39. A method according to claim 38 wherein the presence of the *Ureaplasma urealytica* is determined by determining the presence of *Ureaplasma urealytica* nucleic acid using *Ureaplasma urealytica* specific primers

40. A method according to claim 39 wherein the *Ureaplasma urealytica* specific primers amplify all or part of the urease gene.

41. A method according to claim 40 wherein the primers are:
U1 - 5' GATGGTAAGTTAGTTGCTGAC 3'
U2 - 5' ACGACGTCCATAAGCAACT 3'.

42. A kit for detecting persistent infection with an obligate intracellular pathogenic bacterium comprising
one or more primers specific for an obligate intracellular pathogenic bacterium,
reagents for amplifying pathogen specific nucleic acid from a blood sample using the pathogen specific primers;
detection reagents for detecting the products of amplifying the serum sample with the primers and
a specific binding member which binds to a complex comprising one or more host plasma/serum molecules,
wherein the specific binding member is selected from the group consisting of protein A and antibodies which bind to immunoglobulin, serum lipoproteins or lipopolysaccaride binding proteins.

43. A kit according to claim 42 further comprising apparatus for the removal, handling and storage of blood samples.

44. A kit according to claim 43 further comprising means for the removal of blood cells from the blood sample.

45. A kit according to any one of claims 42 to 44 comprising apparatus for isolating and/or purifying DNA for amplification by said primers.

46. A method of determining the efficacy of an anti-microbial compound in treating persistent infection with an obligate intracellular pathogenic bacterium in an individual comprising:
determining the amount or concentration of cells of a persistent form of an obligate intracellular pathogenic bacterium in acellular blood samples obtained from the individual before and after administration of the anti-microbial compound;
wherein said amount or concentration is determined by;
(a) contacting said blood samples with an specific binding member to isolate and/or purify a fraction of said samples,
wherein the specific binding member binds to a complex comprising one or more host plasma/serum molecules,
wherein the specific binding member is selected from the group consisting of protein A and an antibody which binds to immunoglobulin, serum lipoproteins or lipopolysaccaride binding proteins, and the isolated and/or purified fraction comprises one or more said complexes, and
(b) determining the presence of nucleic acid from the pathogenic bacterium in said fractions of said samples,
wherein a decrease in the amount of nucleic acid from the pathogenic bacterium in said fraction from the sample obtained after administration relative to the sample obtained before administration is indicative that the anti-microbial preparation is efficacious in treating the persistent infection.

## Patentansprüche

1. Verfahren zur Beurteilung eines Individuums bezüglich einer Infektion mit einer persistierenden Form eines obligat intrazellulären pathogenen Bakteriums, Folgendes umfassend:
das Kontaktieren einer zellfreien Blutprobe vom Individuum mit einem spezifischen Bindeelement, um eine Fraktion der Probe zu isolieren und/oder zu reinigen,
worin das spezifische Bindeelement an einen Komplex bindet, der ein oder mehrere Plasma-/Serum-Moleküle des Wirts umfasst, die aus der aus Antikörpern, Serumlipoproteinen oder Lipopolysaccharid-Bindungsproteinen bestehenden Gruppe ausgewählt sind, und die isolierte und/oder gereinigte Fraktion einen oder mehrere der Komplexe umfasst und
worin das spezifische Bindeelement aus der aus Protein A und Antikörpern, die an Immunglobulin, Serumlipoproteine oder Lipopolysaccharid-Bindungsproteine binden, bestehenden Gruppe ausgewählt ist, und
das Bestimmen der Gegenwart von Nucleinsäure vom pathogenen Bakterium in der Fraktion.

2. Verfahren nach Anspruch 1, umfassend:
das Aufkonzentrieren und/oder Extrahieren von DNA aus der isolierten und/oder gereinigten Fraktion und
das Bestimmen der Gegenwart einer Pathogen-Nucleinsäure in der aufkonzentrierten und/oder extrahierten Nucleinsäure.

3. Verfahren nach Anspruch 1 oder Anspruch 2, umfassend das Entfernen von Wirts-Blutzellen aus einer Blutprobe, um die zellfreie Blutprobe herzustellen.

4. Verfahren nach einem der Ansprüche 1 bis 3, worin das eine oder die mehreren Plasma-/Serum-Moleküle Antikörper sind und der Komplex ein Immunkomplex ist.

5. Verfahren nach Anspruch 4, worin das spezifische Bindeelement an Immunglobulin bindet.

6. Verfahren nach Anspruch 5, worin das spezifische Bindeelement Protein A ist.

7. Verfahren nach einem der Ansprüche 1 bis 3, worin die Plasma-/Serum-Moleküle Serumlipoproteine oder Lipopolysaccharid-Bindungsproteine sind.

8. Verfahren nach Anspruch 7, worin das spezifische Bindeelement an die Serumlipoproteine oder Lipopolysaccharid-Bindungsproteine bindet.

9. Verfahren nach einem der vorangegangenen Ansprüche, worin die Gegenwart der Pathogen-Nucleinsäure in der Fraktion das Vorhandensein einer Infektion mit dem Pathogen beim Individuum anzeigt.

10. Verfahren nach Anspruch 9, worin die Gegenwart der Pathogen-Nucleinsäure in der Fraktion das Vorhandensein einer Störung im Zusammenhang mit einer Infektion mit dem Pathogen anzeigt.

11. Verfahren nach Anspruch 10, worin die Gegenwart einer C.-pneumoniae-Nucleinsäure das Vorhandensein eines atherosklerotischen Leidens beim Individuum anzeigt.

12. Verfahren nach Anspruch 10, worin die Gegenwart einer C.-trachomatis-Nucleinsäure das Vorhandensein einer Reproduktionsstörung beim Individuum anzeigt.

13. Verfahren nach Anspruch 10, worin die Gegenwart einer C.-trachomatis-Nucleinsäure das Vorhandensein einer Infektion anzeigt, die aus der aus Vaginitis, Zervizitis und Urethritis bestehenden Gruppe ausgewählt ist.

14. Verfahren nach Anspruch 10, worin die Gegenwart einer C.-trachomatis-Nucleinsäure das Vorhandensein von reaktiver Arthritis (Reiter-Krankheit) anzeigt.

15. Verfahren nach Anspruch 10, worin die Gegenwart einer C.-trachomatis-Nucleinsäure das Vorhandensein einer chronischen Beckenentzündungserkrankung anzeigt.

16. Verfahren nach Anspruch 10, worin die Gegenwart einer C.-trachomatis-Nucleinsäure das Vorhandensein einer asymptomatischen Infektion anzeigt.

17. Verfahren nach Anspruch 10, worin die Gegenwart einer Mycoplasmatacae-Nucleinsäure das Vorhandensein einer Atmungserkrankung, Beckenentzündungserkrankung oder Reproduktionsstörung beim Individuum anzeigt.

18. Verfahren nach einem der vorangegangenen Ansprüche, worin die Gegenwart von Pathogen-Nucleinsäure in der isolierten und/oder gereinigten Fraktion durch Amplifikation mit pathogenspezifischen Primer bestimmt wird,
wobei die Gegenwart von Amplifikationsprodukten die Gegenwart von Pathogen-Nucleinsäure anzeigt.

19. Verfahren nach Anspruch 18, worin die Gegenwart von Pathogen-Nucleinsäure in der Probe durch PCR unter Verwendung von pathogenspezifischen Primern bestimmt wird.

20. Verfahren nach einem der vorangegangenen Ansprüche, worin das Pathogen eine Chlamydia spp. ist.

21. Verfahren nach Anspruch 20, worin die Chlamydia spp. C. pneumoniae oder C. trachomatis ist.

22. Verfahren nach Anspruch 20 oder Anspruch 21, worin die Gegenwart der Chlamydien-Zelle durch Bestimmen der Gegenwart von Chlamydien-Nucleinsäure unter Verwendung von Chlamydien-spezifischen Primern bestimmt wird.

23. Verfahren nach Anspruch 22, worin die Chlamydienspezies Chlamydia pneumoniae ist.

24. Verfahren nach Anspruch 23, worin die Chlamydien-spezifischen Primer das gesamte 16S-RNA-Gen von Chlamydia pneumoniae oder einen Teil davon amplifizieren.

25. Verfahren nach Anspruch 24, worin die Primer aus folgender Gruppe ausgewählt sind:
5' GGT CTC AAC CCC ATC T 3'
5'-GGT CTC AAC CCC ATC CGT GTC GG-3'
5' TGC GGA AAG CTG TAT TTC TAC AGT T 3'
5'-CAAGTCCAGGTAAGGTCCTTCGCGTTGC-3' und
5'-TCCAGGTAAGGTCCTTCGCGTTGCATCG-3'.

26. Verfahren nach Anspruch 23, worin die Chlamydien-spezifischen Primer das gesamte OmpA-Gen von Chlamydia pneumoniae oder einen Teil davon amplifizieren.

27. Verfahren nach Anspruch 26, worin die Primer aus folgender Gruppe ausgewählt sind:
5' CCA ATA TGC ACA GTC CAA ACC TAA AA 3'
5' CTA GAT TTA AAC TTG TTG ATC TGA CAG 3'
5' CTC TGT AAA CAA ACC GGG C 3'
5' GAT CTG ACA GGA AAC AAT TTG CAT 3'.

28. Verfahren nach Anspruch 22, worin das Pathogen Chlamydia trachomatis ist.

29. Verfahren nach Anspruch 28, worin die Chlamydia-trachomatis-spezifischen Primer das gesamte kryptische C.-trachomatis-Plasmid oder einen Teil davon amplifizieren.

30. Verfahren nach Anspruch 29, worin die Primer wie folgt sind:
CP 24 - 5' GGGATTCCTGTAACAACAAGTCAGG 3'
CP 27 - 5' CCTCTTCCCCAGAACAATAAGAAC 3'.

31. Verfahren nach einem der Ansprüche 1 bis 19, worin das Pathogen eine Mycoplasmataceae-Spezies ist.

32. Verfahren nach Anspruch 31, worin das Pathogen ein Mycoplasma ist.

33. Verfahren nach Anspruch 32, worin das Mycoplasma M. genitalium ist.

34. Verfahren nach Anspruch 33, worin die Gegenwart von Mycoplasma genitalium durch Bestimmen der Gegenwart von Mycoplasma-genitalium-Nucleinsäure unter Verwendung von Mycoplasma-genitalium-spezifischen Primern bestimmt wird.

35. Verfahren nach Anspruch 34, worin die Mycoplasma-genitalium-spezifischen Primer das gesamte MgPa-Gen oder einen Teil davon amplifizieren.

36. Verfahren nach Anspruch 35, worin die Primer wie folgt sind:
MgPa-1 - 5' AGTTGATGAAACCTTAACCCCTTGG 3'
MgPa-3 - 5' CCGTTGAGGGGTTTTCCATTTTTGC 3'.

37. Verfahren nach Anspruch 31, worin das Pathogen ein Ureaplasma ist.

38. Verfahren nach Anspruch 37, worin das Ureaplasma U. urealyticum ist.

39. Verfahren nach Anspruch 38, worin die Gegenwart von Ureaplasma urealytica durch Bestimmen der Gegenwart von Ureaplasma-urealytica-Nucleinsäure unter Verwendung von Ureaplasma-urealytica-spezifischen Primern bestimmt wird.

40. Verfahren nach Anspruch 39, worin die Ureaplasma-urealytica-spezifischen Primer das gesamte Urease-Gen oder einen Teil davon amplifizieren.

41. Verfahren nach Anspruch 40, worin die Primer wie folgt sind:
U 1 - 5' GATGGTAAGTTAGTTGCTGAC 3'
U2 - 5' ACGACGTCCATAAGCAACT 3'.

42. Set zur Detektion einer persistierenden Infektion mit einem obligat intrazellulären pathogenen Bakterium, umfassend:
einen oder mehrere Primer, die für ein obligat intrazelluläres pathogenes Bakterium spezifisch sind,
Reagenzien zur Amplifikation von pathogenspezifischer Nucleinsäure aus einer Blutprobe unter Verwendung der pathogenspezifischen Primer,
Detektionsreagenzien zur Detektion von Produkten der Amplifikation der Serumprobe mit den Primern und
ein spezifisches Bindeelement, das an einen Komplex bindet, der ein oder mehrere Plasma-/Serum-Moleküle des Wirts umfasst,
worin das spezifische Bindeelement aus der aus Protein A und Antikörpern, die an Immunglobulin, Serumlipoproteine oder Lipopolysaccharid-Bindungsproteine binden, bestehenden Gruppe ausgewählt ist.

43. Set nach Anspruch 42, das weiters ein Gerät zur Entfernung, Handhabung und Lagerung von Blutproben umfasst.

44. Set nach Anspruch 43, das weitere Mittel zur Entfernung von Blutzellen aus der Blutprobe umfasst.

45. Set nach einem der Ansprüche 42 bis 44, das ein Gerät zur Isolierung und/oder Reinigung von DNA zur Amplifikation durch die genannten Primer umfasst.

46. Verfahren zur Bestimmung der Wirksamkeit einer antimikrobiellen Verbindung bei der Behandlung einer persistierenden Infektion mit einem obligat intrazellulären pathogenen Bakterium bei einem Individuum, Folgendes umfassend:
das Bestimmen der Menge oder Konzentration von Zellen einer persistierenden Form eines obligat intrazellulären pathogenen Bakteriums in zellfreien Blutproben, die dem Individuum vor und nach Verabreichung der antimikrobiellen Verbindung abgenommen wurden;
worin die Menge oder Konzentration wie folgt bestimmt wird:
(a) Kontaktieren der Blutproben mit einem spezifischen Bindeelement, um eine Fraktion der Proben zu isolieren oder zu reinigen,
worin das spezifische Bindeelement an einen Komplex bindet, der ein oder mehrere Plasma-/Serum-Moleküle des Wirts umfasst,
worin das spezifische Bindeelement aus der aus Protein A und einem Antikörper, der an Immunglobulin, Serumlipoproteine oder Lipopolysaccharid-Bindungsproteine bindet, bestehenden Gruppe ausgewählt ist und die isolierte und/oder gereinigte Fraktion einen oder mehrere der Komplexe umfasst, und
(b) Bestimmen der Gegenwart von Nucleinsäure vom pathogenen Bakterium in den Fraktionen der Proben,
worin eine Verringerung der Menge an Nucleinsäure vom pathogenen Bakterium in der Fraktion der Probe, die nach der Verabreichung erhalten wurde, im Vergleich zur Probe, die vor der Verabreichung erhalten wurde, anzeigt, dass das antimikrobielle Präparat wirksam zur Behandlung der persistierenden Infektion ist.

## Revendications

1. Procédé pour évaluer la présence d'une infection par une forme persistante de bactérie pathogène intracellulaire obligatoire chez un individu, consistant à :
mettre en contact un échantillon de sang acellulaire de l'individu avec un membre de liaison spécifique afin d'isoler et/ou de purifier une fraction dudit échantillon,
où le membre de liaison spécifique se lie à un complexe comprenant une ou plusieurs molécules plasmatiques/sériques de l'hôte sélectionnées dans le groupe consistant en des anticorps, des lipoprotéines sériques ou des protéines de liaison aux polysaccharides, et la fraction isolée et/ou purifiée comprend un ou plusieurs desdits complexes, et
où le membre de liaison spécifique est sélectionné dans le groupe consistant en la protéine A et des anticorps qui se lient à une immunoglobuline, des lipoprotéines sériques ou des protéines de liaison aux polysaccharides, et ;
déterminer la présence d'un acide nucléique de la bactérie pathogène dans ladite fraction.

2. Procédé selon la revendication 1, consistant à :
concentrer et/ou extraire de l'ADN à partir de la fraction isolée et/ou purifiée, et ;
déterminer la présence d'un acide nucléique pathogène dans l'acide nucléique concentré et/ou extrait.

3. Procédé selon la revendication 1 ou la revendication 2, consistant à éliminer les cellules sanguines de l'hôte d'un échantillon de sang afin de produire l'échantillon de sang acellulaire.

4. Procédé selon l'une quelconque des revendications 1 à 3, dans lequel la une ou plusieurs molécules de plasmatiques/sériques sont des anticorps et le complexe est un complexe immunologique.

5. Procédé selon la revendication 4, dans lequel le membre de liaison spécifique se lie à une immunoglobuline.

6. Procédé selon la revendication 5, dans lequel le membre de liaison spécifique est la protéine A.

7. Procédé selon l'une quelconque des revendications 1 à 3, dans lequel les molécules plasmatiques/sériques sont des lipoprotéines sériques ou des protéines de liaison aux lipopolysaccharides.

8. Procédé selon la revendication 7, dans lequel le membre de liaison spécifique se lie auxdites lipoprotéines sériques ou protéines de liaison aux lipopolysaccharides.

9. Procédé selon l'une quelconque des revendications précédentes, dans lequel la présence de l'acide nucléique pathogène dans ladite fraction est indicatrice de la présence de l'infection par un agent pathogène chez l'individu.

10. Procédé selon la revendication 9, dans lequel la présence de l'acide nucléique pathogène dans ladite fraction est indicatrice de la présence d'un trouble associé à une infection par un agent pathogène.

11. Procédé selon la revendication 10, dans lequel la présence d'un acide nucléique de *C*. *pneumoniae* est indicatrice de la présence d'un état athérosclérotique chez l'individu.

12. Procédé selon la revendication 10, dans lequel la présence d'un acide nucléique de *C*. *trachomatis* est indicatrice de la présence d'un dysfonctionnement de la reproduction chez l'individu.

13. Procédé selon la revendication 10, dans lequel la présence d'un acide nucléique de *C*. *trachomatis* est indicatrice de la présence d'une infection sélectionnée dans le groupe consistant en une vaginite, une cervicite et une urétrite.

14. Procédé selon la revendication 10, dans lequel la présence d'un acide nucléique de *C*. *trachomatis* est indicatrice de la présence d'une arthrite réactionnelle (maladie de Reiter).

15. Procédé selon la revendication 10, dans lequel la présence d'un acide nucléique de *C*. *trachomatis* est indicatrice de la présence d'une maladie inflammatoire pelvienne chronique.

16. Procédé selon la revendication 10, dans lequel la présence d'un acide nucléique de *C*. *trachomatis* est indicatrice de la présence d'une infection asymptomatique.

17. Procédé selon la revendication 10, dans lequel la présence d'un acide nucléique de *Mycoplasmataceae* est indicatrice de la présence d'un trouble respiratoire, d'une maladie inflammatoire pelvienne ou d'un dysfonctionnement de la reproduction chez l'individu.

18. Procédé selon l'une quelconque des revendications précédentes, dans lequel la présence d'un acide nucléique pathogène dans la fraction isolée et/ou purifiée est déterminée par une amplification avec des amorces spécifiques à l'agent pathogène,
la présence de produits d'amplification étant indicatrice de la présence d'un acide nucléique pathogène.

19. Procédé selon la revendication 18, dans lequel la présence d'un acide nucléique pathogène dans l'échantillon est déterminée par PCR en utilisant des amorces spécifiques à un agent pathogène.

20. Procédé selon l'une quelconque des revendications précédentes, dans lequel l'agent pathogène est une espèce Chlamydia.

21. Procédé selon la revendication 20, dans lequel l'espèce Chlamydia est *C*. *pneumoniae* ou *C*. *trachomatis.*

22. Procédé selon la revendication 20 ou la revendication 21, dans lequel la présence de la cellule de Chlamydia est déterminée en déterminant la présence d'un acide nucléique de Chlamydia en utilisant des amorces spécifique à Chlamydia.

23. Procédé selon la revendication 22, dans lequel la Chlamydia est *Chlamydia pneumoniae.*

24. Procédé selon la revendication 23, dans lequel les amorces spécifiques à Chlamydia amplifient l'intégralité ou une partie du gène de l'ARN 16S de *Chlamydia pneumoniae.*

25. Procédé selon la revendication 24, dans lequel les amorces sont sélectionnées dans le groupe consistant en :
5' GGT CTC AAC CCC ATC T 3'
5'-GGT CTC AAC CCC ATC CGT GTC GG-3'
5' TGC GGA AAG CTG TAT TTC TAC AGT T 3'
5'-CAAGTCCAGGTAAGGTCCTTCGCGTTGC-3' et,
5'-TCCAGGTAAGGTCCTTCGCGTTGCATCG-3'

26. Procédé selon la revendication 23, dans lequel les amorces spécifiques à Chlamydia amplifient l'intégralité ou une partie du gène OmpA de *Chlamydia pneumoniae.*

27. Procédé selon la revendication 26, dans lequel les amorces sont sélectionnées dans le groupe consistant en :
5' CCA ATA TGC ACA GTC CAA ACC TAA AA 3'
5' CTA GAT TTA AAC TTG TTG ATC TGA CAG 3'
5' CTC TGT AAA CAA ACC GGG C 3'
5' GAT CTG ACA GGA AAC AAT TTG CAT 3'

28. Procédé selon la revendication 22, dans lequel l'agent pathogène est *Chlamydia trachomatis.*

29. Procédé selon la revendication 28, dans lequel les amorces spécifiques à *Chlamydia trachomatis* amplifient l'intégralité ou une partie du plasmide cryptique de *C*. *trachomatis.*

30. Procédé selon la revendication 29, dans lequel les amorces sont :
CP 24 - 5' GGGATTCCTGTAACAACAAGTCAGG 3'
CP 27 - 5' CCTCTTCCCCAGAACAATAAGAAC 3'.

31. Procédé selon l'une quelconque des revendications 1 à 19, dans lequel l'agent pathogène est une espèce *Mycoplasmataceae.*

32. Procédé selon la revendication 31, dans lequel l'agent pathogène est un mycoplasma.

33. Procédé selon la revendication 32, dans lequel le mycoplasma est *M. genitalium.*

34. Procédé selon la revendication 33, dans lequel la présence de *Mycoplasma genitalium* est déterminée en déterminant la présence d'un acide nucléique de *Mycoplasma genitalium* en utilisant des amorces spécifiques à *Mycoplasma genitalium.*

35. Procédé selon la revendication 34, dans lequel les amorces spécifiques à *Mycoplasma genitalium* amplifient l'intégralité ou une partie du gène MgPa.

36. Procédé selon la revendication 35, dans lequel les amorces sont :
MgPa-1 - 5' AGTTGATGAAACCTTAACCCCTTGG 3'
MgPa-3 - 5' CCGTTGAGGGGTTTTCCATTTTTGC 3'

37. Procédé selon la revendication 31, dans lequel l'agent pathogène est un ureaplasma.

38. Procédé selon la revendication 37, dans lequel l'ureaplasma est *U. urealyticum.*

39. Procédé selon la revendication 38, dans lequel la présence de *Ureaplasma urealytica* est déterminée en déterminant la présence d'un acide nucléique de *Ureaplasma urealytica* en utilisant des amorces spécifiques à *Ureaplasma urealytica.*

40. Procédé selon la revendication 39, dans lequel les amorces spécifiques à *Ureaplasma urealytica* amplifient l'intégralité ou une partie du gène de l'uréase.

41. Procédé selon la revendication 40, dans lequel les amorces sont :
U1 - 5' GATGGTAAGTTAGTTGCTGAC 3'
U2 - 5' ACGACGTCCATAAGCAACT 3'.

42. Kit pour détecter une infection persistante par une bactérie pathogène intracellulaire obligatoire, comprenant
une ou plusieurs amorces spécifiques à une bactérie pathogène intracellulaire obligatoire,
des réactifs pour amplifier un acide nucléique spécifique à l'agent pathogène à partir d'un échantillon de sang en utilisant les amorces spécifiques à l'agent pathogène ;
des réactifs de détection pour détecter les produits d'amplification de l'échantillon de sérum avec les amorces et
un membre de liaison spécifique qui se lie à un complexe comprenant une ou plusieurs molécules plasmatiques/sériques de l'hôte,
où le membre de liaison spécifique est sélectionné dans le groupe consistant en la protéine A et des anticorps qui se lient à une immunoglobuline, des lipoprotéines sériques ou des protéines de liaison aux polysaccharides.

43. Kit selon la revendication 42, comprenant en outre un appareil pour le prélèvement, la manipulation et le stockage des échantillons de sang.

44. Kit selon la revendication 43, comprenant en outre un moyen pour l'élimination des cellules sanguines à partir de l'échantillon de sang.

45. Kit selon l'une quelconque des revendications 42 à 44, comprenant un appareil pour isoler et/ou purifier de l'ADN pour une amplification par lesdites amorces.

46. Procédé pour déterminer l'efficacité d'un composé antimicrobien dans le traitement d'une infection persistante par une bactérie pathogène intracellulaire obligatoire chez un individu, consistant à :
déterminer la quantité ou la concentration de cellules d'une forme persistante de bactérie pathogène intracellulaire obligatoire dans des échantillons de sang acellulaire obtenus à partir de l'individu avant et après l'administration du composé antimicrobien ;
où ladite quantité ou concentration est déterminée par :
(a) la mise en contact desdits échantillons de sang avec un membre de liaison spécifique afin d'isoler et/ou de purifier une fraction desdits échantillons,
où le membre de liaison spécifique se lie à un complexe comprenant une ou plusieurs molécules plasmatiques/sériques de l'hôte,
où le membre de liaison spécifique est sélectionné dans le groupe consistant en la protéine A et un anticorps qui se lie à une immunoglobuline, des lipoprotéines sériques ou des protéines de liaison aux lipopolysaccharides, et la fraction isolée et/ou purifiée comprend un ou plusieurs desdits complexes, et
(b) déterminer la présence d'un acide nucléique de la bactérie pathogène dans lesdites fractions desdits échantillons,
où une réduction de la quantité d'acide nucléique de la bactérie pathogène dans ladite fraction de l'échantillon obtenu après l'administration, par rapport à l'échantillon obtenu avant l'administration, indique que la préparation antimicrobienne est efficace pour traiter l'infection persistante.
